# EUROPEAN PATENT APPLICATION

(11) **EP 1 002 862 A1**
(43) Date of publication of application: **24.05.2000**
(21) Application number: 98121478.6
(22) Date of filing: 12.11.1998
(51) Int. Cl.: C12N 15/12, C07K 14/47, C12Q 1/68, G01N 33/68, A61K 38/17

(54) **Methods of diagnosing or treating neurological diseases**

(71) Applicant: Nitsch, Roger M., Prof. Dr., 20146 Hamburg (DE)
(72) Inventor: The designation of the inventor has not yet been filed
(74) Representative: Meyers, Hans-Wilhelm, Dr.Dipl.-Chem.

(57) **Abstract**

An isolated nucleic acid molecule encoding a protein molecule shown in SEQ ID No. 1.

SEQUENCE LISTING

(1) GENERAL INFORMATION:

(i) APPLICANT:
(A) NAME: Prof. Dr. Roger M. Nitsch
(B) STREET: Hartungstrasse 8
(C) CITY: Hamburg
(E) COUNTRY: Germany
(F) POSTAL CODE (ZIP): D-20146

(ii) TITLE OF INVENTION: Methods of diagnosing or treating
neurological diseases

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 516 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO: 1:

(2) INFORMATION FOR SEQ ID NO: 2:

(i) SEQUENCE CHARACTERISTICS:
(A) LENGTH: 4248 base pairs
(B) TYPE: nucleic acid
(C) STRANDEDNESS: single
(D) TOPOLOGY: unknown

(ii) MOLECULE TYPE: cDNA

(xi) SEQUENCE DESCRIPTION: SEQ ID NO: 2:

CCCGGGCTGT GGGCTACAGG CGCAGAGCGG GCCAGGCGCG GAGCTGGCGG CAGTGACAGG 60

AGGCGCGAAC CCGCAGCGCT TACCGCGCGG CGCCGCACCA TGGAGCCCGC CGTGTCGCTG 120

GCCGTGTGCG CGCTGCTCTT CCTGCTGTGG GTGCGCCTGA AGGGGCTGGA GTTCGTGCTC 180

ATCCACCAGC GCTGGGTGTT CGTGTGCCTC TTCCTCCTGC CGCTCTCGCT TATCTTCGAT 240

ATCTACTACT ACGTGCGCGC CTGGGTGGTG TTCAAGCTCA GCAGCGCTCC GCGCCTGCAC 300

GAGCAGCGCG TGCGGGACAT CCAGAAGCAG GTGCGGGAAT GGAAGGAGCA GGGTAGCAAG 360

ACCTTCATGT GCACGGGGCG CCCTGGCTGG CTCACTGTCT CACTACGTGT CGGGAAGTAC 420

AAGAAGACAC ACAAAAACAT CATGATCAAC CTGATGGACA TTCTGGAAGT GGACACCAAG 480

AAACAGATTG TCCGTGTGGA GCCCTTGGTG ACCATGGGCC AGGTGACTGC CCTGCTGACC 540

TCCATTGGCT GGACTCTCCC CGTGTTGCCT GAGCTTGATG ACCTCACAGT GGGGGGCTTG 600

ATCATGGGCA CAGGCATCGA GTCATCATCC CACAAGTACG GCCTGTTCCA ACACATCTGC 660

ACTGCTTACG AGCTGGTCCT GGCTGATGGC AGCTTTGTGC GATGCACTCC GTCCGAAAAC 720

TCAGACCTGT TCTATGCCGT ACCCTGGTCC TGTGGGACGC TGGGTTTCCT GGTGGCCGCT 780

GAGATCCGCA TCATCCCTGC CAAGAAGTAC GTCAAGCTGC GTTTCGAGCC AGTGCGGGGC 840

CTGGAGGCTA TCTGTGCCAA GTTCACCCAC GAGTCCCAGC GGCAGGAGAA CCACTTCGTG 900

GAAGGGCTGC TCTACTCCCT GGATGAGGCT GTCATTATGA CAGGGGTCAT GACAGATGAG 960

GCAGAGCCCA GCAAGCTGAA TAGCATTGGC AATTACTACA AGCCGTGGTT CTTTAAGCAT 1020

GTGGAGAACT ATCTGAAGAC AAACCGAGAG GGCCTGGAGT ACATTCCCTT GAGACACTAC 1080

TACCACCGCC ACACGCGCAG CATCTTCTGG GAGCTCCAGG ACATCATCCC CTTTGGCAAC 1140

AACCCCATCT TCCGCTACCT CTTTGGCTGG ATGGTGCCTC CCAAGATCTC CCTCCTGAAG 1200

CTGACCCAGG GTGAGACCCT GCGCAAGCTG TACGAGCAGC ACCACGTGGT GCAGGACATG 1260

CTGGTGCCCA TGAAGTGCCT GCAGCAGGCC CTGCACACCT TCCAAAACGA CATCCACGTC 1320

TACCCCATCT GGCTGTGTCC GTTCATCCTG CCCAGCCAGC CAGGCCTAGT GCACCCCAAA 1380

GGAAATGAGG CAGAGCTCTA CATCGACATT GGAGCATATG GGGAGCCGCG TGTGAAACAC 1440

TTTGAAGCCA GGTCCTGCAT GAGGCAGCTG GAGAAGTTTG TCCGCAGCGT GCATGGCTTC 1500

CAGATGCTGT ATGCCGACTG CTACATGAAC CGGGAGGAGT TCTGGGAGAT GTTTGATGGC 1560

TCCTTGTACC ACAAGCTGCG AGAGAAGCTG GGTTGCCAGG ACGCCTTCCC CGAGGTGTAC 1620

GACAAGATCT GCAAGGCCGC CAGGCACTGA GCTGGAGCCC GCCTGGAGAG ACAGACACGT 1680

GTGAGTGGTC AGGCATCTTC CCTTCACTCA AGCTTGGCTG CTTTCCTAGA TCCACACTTT 1740

CAAAGAGAAA CCCCTCCAGA ACTCCCACCC TGACAGCCCA ACACCACCTT CCTCCTGGCT 1800

TCCAGGGGGC AGCCCAGTGG AATGGAAAGA ATGTGGGATT TGGAGTCAGA CAAGCCTGAG 1860

TCCAGTTCCC CGTTTAGAAC TCATTAGCTG TGTGACTCTG GGTGAGTCCC TTAACCCCTC 1920

TGAGCCCGGG TCTCTTCATT AGTTGAAAGG GATAGTAATA CCTACTTGCA GGTTGTTGTC 1980

ATCTGAGTTG AGCACTGGTC ACATTGAAGG TGCTGGGTAA GTGGTAGCTC TTGTTGCTTC 2040

CCGTTCAGCG TCACATCTGC AGTGGAGCCT GAAAAGGCTC CACATTAGGT CACCTGTGCA 2100

CAGCCATGGC TGGAATGATG AAGGGGATAC GCTGGAGTTG CCCTGCCATC GCCTCCATCA 2160

GCCAGACGAG GTCCTCACAG GAGAAGGACA GCTCTTCCCC ACCCTGGGAT CTCAGGAGGG 2220

CAGCCACGGA GTGGGGAGGC CCCAGATGCG CTGTGCCAAA GCCAGGTCCG AGGCCAAAGT 2280

TCTCCCTGCC ATCCTTGGTG CCGTCCTGCC CCTTCCTCCT TCATGCCTGG GCCTGCAGGC 2340

CCACCCCAGC CACCACTGAG TCCACTCGGA GTGCCCTGTG TTCCTGGAGA AGGCATTCCA 2400

GGGTTGAATC TTGTCCCAGC CTCAGCCTGG GACACCTAGG TGGAGAGAGT GGTCTCCGCT 2460

CTGAATTGGA TCCAGGGGAC CTGGGCTCAT TCTTCTTGGC TCACCAACCC TGCAGGCCTC 2520

ATCTTTCCCA AAACCCACTT TGTCTTGGTG GGAGTGGGTC CGCGCTGCTC TGCAGCAGGG 2580
GCTGGGGAGT GGACAGCATC AGGTGGGAAA GTGGAGTCCA CCCTCATGTT TCTGTAGGAT 2640
TCTCACCGTG GGGCTGGAAG AAAAGAGCAT CGACTTGATT TCTCCAACCA CTCATCCCTC 2700
TTTTTCTTTC TTCCACCACT CCCCACCCCA GCTGTAGTTA ATTTCAGTGC CTTACAAATC 2760
CTAAGCTCAG AGAAAGTTCC ATTTCCGTTC CAGAGGGAAG GGAACCTCCC TAGGTCCTTC 2820
CCTGGCTTGT TATAACGCAA AGCTTGGTTG TTTATGCAAC TCTATCTTAA GAACTGCCCA 2880
GCCTCAGCTG AAAACCCGAA TCTGAGAAGG AATTGCGTCA TGTAAGGGAA GCTGGAATTA 2940
AGGGAGCTGA GCCAGTCATG GTTGTGGCGT GTGAGTCAGG AGACCTAGGT TTCAGCCCCT 3000
CTCTACTGTC AGCGAGCTGT GCAACGTGGG CAAGTCATTG TCCTCTGAGC TGCAGTTTCC 3060
TCATCTGTCA CATCGCTACA GACAAGACCT CCCTGGAACC CTTCTGATTG TCTTAGACAC 3120
TGTGGTTGCA AAACCCACGG AAAGCCTCAT TTGTGTGGAA AGTCAGAGGA AAAATGATCC 3180
AGTGGACACT TGGGGATTAT CTGTCATTCA AGATCCTTCC TTCAACCCCA AGGCCAGCTC 3240
CCATCTCATT TCCAGAAAGG CTCATACCTG GCTTGCAGGG AAGCATCTGT CTTGTCATTC 3300
CAGGTGCCAG AATCCTCTCA GAGTCATTGA AGGGTGTTCA CCCATCCCAC CCAAGGCTTG 3360
GCACACTGCC AGTGTCTTAG CAGGGTCTTG TGAGGGCTGG GGGCATCCAG GCACTCAGAA 3420
GGCAAAGGAA CCACCCTACC CATTTGGCCT CTGGAGGGGG CAGAAGAAAG AAAGAAACCT 3480
CATCCTATAT TTTACAAAGC ATGTGAATTC TGGCATTAGC TCTCATAGGA GACCCATGTG 3540
CTTCCTTGCT CAGTGCAAAA CTGATGATTC TACTTGCTGT AGATGAATGG TTAACACGAG 3600
CTAGTTAAAC AGTGCCATTG TTTTGCCAGT GAAGCCTCCA ACCCTAAGCC ACTGGGACGG 3660
TGGCCAGAGA TGCCAGCAGC CTCTGTCGCC CTTAGTCATA TAACCAAAAT CCAGACCTTA 3720
TCCACAACCC GGGGCTTGGA AAGGAAGGTA TTTTGGAATC ACACCCTCCG GTTATGTTGC 3780
TCCAGTAAAA TCTTGCCTGG AAAGAGGCAG TCTTCTTAGC ATGGTGAGCT GAGTTCATGG 3840
CTTTTTTTTG TAGCCAGTCC TGTCCCTGGC CATCCATGTG ATGGTTTTGG ATGGAGTTAA 3900
ACTTGATGCC AGTGGGCAGT GCATGTGGAA AGTATCAGAG TAAGCCTCTC CCCTCCAGAG 3960
CCCTGAGTTT CTTGGCTGCA TGAAGGTTTT CTTTAGAATC AGAATTGTAG CCAGTTTCTT 4020
TGGCCAGAAG GATGAATACT TGGATATTAC TGAAAGGGAG GGGTGGAGAT GGGTGTGGCA 4080
GTGTATGGTG TGTGATTTTT ATTTTCTTCT TTGGTCATGG GGGCCAAGGA GAAAGGCATG 4140
AATCTTCCCT GTCAGGCTCT TACAGCCACA GGCACTGTGT CTACTGTCTG GAAGACATGT 4200
CCCCGTGGCT GTGGGGCCGC TGCTTCTGTT TAAATAAAAG TGGCCTGG 4248

(2) INFORMATION FOR SEQ ID NO: 3:

(i) SEQUENCE CHARACTERISTICS:
(A) LENGTH: 4187 base pairs
(B) TYPE: nucleic acid
(C) STRANDEDNESS: unknown
(D) TOPOLOGY: unknown

(ii) MOLECULE TYPE: cDNA

(xi) SEQUENCE DESCRIPTION: SEQ ID NO: 3:

GGCGCGAACC CGCAGCGCTT ACCGCGCGGC GCCGCACCAT GGAGCCCGCC GTGTCGCTGG 60

CCGTGTGCGC GCTGCTCTTC CTGCTGTGGG TGCGCCTGAA GGGGCTGGAG TTCGTGCTCA 120

TCCACCAGCG CTGGGTGTTC GTGTGCCTCT TCCTCCTGCC GCTCTCGCTT ATCTTCGATA 180

TCTACTACTA CGTGCGCGCC TGGGTGGTGT TCAAGCTCAG CAGCGCTCCG CGCCTGCACG 240

AGCAGCGCGT GCGGGACATC CAGAAGCAGG TGCGGGAATG GAAGGAGCAG GGTAGCAAGA 300

CCTTCATGTG CACGGGGCGC CCTGGCTGGC TCACTGTCTC ACTACGTGTC GGGAAGTACA 360

AGAAGACACA CAAAAACATC ATGATCAACC TGATGGACAT TCTGGAAGTG GACACCAAGA 420

AACAGATTGT CCGTGTGGAG CCCTTGGTGA CCATGGGCCA GGTGACTGCC CTGCTGACCT 480

CCATTGGCTG GACTCTCCCC GTGTTGCCTG AGCTTGATGA CCTCACAGTG GGGGGCTTGA 540

TCATGGGCAC AGGCATCGAG TCATCATCCC ACAAGTACGG CCTGTTCCAA CACATCTGCA 600

CTGCTTACGA GCTGGTCCTG GCTGATGGCA GCTTTGTGCG ATGCACTCCG TCCGAAAACT 660

CAGACCTGTT CTATGCCGTA CCCTGGTCCT GTGGGACGCT GGGTTTCCTG GTGGCCGCTG 720

AGATCCGCAT CATCCCTGCC AAGAAGTACG TCAAGCTGCG TTTCGAGCCA GTGCGGGGCC 780

TGGAGGCTAT CTGTGCCAAG TTCACCCACG AGTCCCAGCG GCAGGAGAAC CACTTCGTGG 840

AAGGGCTGCT CTACTCCCTG GATGAGGCTG TCATTATGAC AGGGGTCATG ACAGATGAGG 900

CAGAGCCCAG CAAGCTGAAT AGCATTGGCA ATTACTACAA GCCGTGGTTC TTTAAGCATG 960

TGGAGAACTA TCTGAAGACA AACCGAGAGG GCCTGGAGTA CATTCCCTTG AGACACTACT 1020

ACCACCGCCA CACGCGCAGC ATCTTCTGGG AGCTCCAGGA CATCATCCCC TTTGGCAACA 1080

ACCCCATCTT CCGCTACCTC TTTGGCTGGA TGGTGCCTCC CAAGATCTCC CTCCTGAAGC 1140

TGACCCAGGG TGAGACCCTG CGC[?]AGCTGT ACGAGCAGCA CCACGTGGTG CAGGACATGC 1200

TGGTGCCCAT GAAGTGCCTG CAGCAGGCCC TGCACACCTT CCAAAACGAC ATCCACGTCT 1260

ACCCCATCTG GCTGTGTCCG TTCATCCTGC CCAGCCAGCC AGGCCTAGTG CACCCCAAAG 1320

GAAATGAGGC AGAGCTCTAC ATCGACATTG GAGCATATGG GGAGCCGCGT GTGAAACACT 1380

TTGAAGCCAG GTCCTGCATG AGGCAGCTGG AGAAGTTTGT CCGCAGCGTG CATGGCTTCC 1440
AGATGCTGTA TGCCGACTGC TACATGAACC GGGAGGAGTT CTGGGAGATG TTTGATGGCT 1500
CCTTGTACCA CAAGCTGCGA GAGAAGCTGG GTTGCCAGGA CGCCTTCCCC GAGGTGTACG 1560
ACAAGATCTG CAAGGCCGCC AGGCACTGAG CTGGAGCCCG CCTGGAGAGA CAGACACGTG 1620
TGAGTGGTCA GGCATCTTCC CTTCACTCAA GCTTGGCTGC TTTCCTAGAT CCACACTTTC 1680
AAAGAGAAAC CCCTCCAGAA CTCCCACCCT GACAGCCCAA CACCACCTTC CTCCTGGCTT 1740
CCAGGGGGCA GCCCAGTGGA ATGGAAAGAA TGTGGGATTT GGAGTCAGAC AAGCCTGAGT 1800
CCAGTTCCCC GTTTAGAACT CATTAGCTGT GTGACTCTGG GTGAGTCCCT TAACCCCTCT 1860
GAGCCCGGGT CTCTTCATTA GTTGAAAGGG ATAGTAATAC CTACTTGCAG GTTGTTGTCA 1920
TCTGAGTTGA GCACTGGTCA CATTGAAGGT GCTGGGTAAG TGGTAGCTCT TGTTGCTTCC 1980
CGTTCAGCGT CACATCTGCA GTGGAGCCTG AAAAGGCTCC ACATTAGGTC ACCTGTGCAC 2040
AGCCATGGCT GGAATGATGA AGGGGATACG CTGGAGTTGC CCTGCCATCG CCTCCATCAG 2100
CCAGACGAGG TCCTCACAGG AGAAGGACAG CTCTTCCCCA CCCTGGGATC TCAGGAGGGC 2160
AGCCACGGAG TGGGGAGGCC CCAGATGCGC TGTGCCAAAG CCAGGTCCGA GGCCAAAGTT 2220
CTCCCTGCCA TCCTTGGTGC CGTCCTGCCC CTTCCTCCTT CATGCCTGGG CCTGCAGGCC 2280
CACCCCAGCC ACCACTGAGT CCACTCGGAG TGCCCTGTGT TCCTGGAGAA GGCATTCCAG 2340
GGTTGAATCT TGTCCCAGCC TCAGCCTGGG ACACCTAGGT GGAGAGAGTG GTCTCCGCTC 2400
TGAATTGGAT CCAGGGGACC TGGGCTCATT CTTCTTGGCT CACCAACCCT GCAGGCCTCA 2460
TCTTTCCCAA AACCCACTTT GTCTTGGTGG GAGTGGGTCC GCGCTGCTCT GCAGCAGGGG 2520
CTGGGGAGTG GACAGCATCA GGTGGGAAAG TGGAGTCCAC CCTCATGTTT CTGTAGGATT 2580
CTCACCGTGG GGCTGGAAGA AAAGAGCATC GACTTGATTT CTCCAACCAC TCATCCCTCT 2640
TTTTCTTTCT TCCACCACTC CCCACCCCAG CTGTAGTTAA TTTCAGTGCC TTACAAATCC 2700
TAAGCTCAGA GAAAGTTCCA TTTCCGTTCC AGAGGGAAGG GAACCTCCCT AGGTCCTTCC 2760
CTGGCTTGTT ATAACGCAAA GCTTGGTTGT TTATGCAACT CTATCTTAAG AACTGCCCAG 2820
CCTCAGCTGA AAACCCGAAT CTGAGAAGGA ATTGCGTCAT GTAAGGGAAG CTGGAATTAA 2880
GGGAGCTGAG CCAGTCATGG TTGTGGCGTG TGAGTCAGGA GACCTAGGTT TCAGCCCCTC 2940
TCTACTGTCA GCGAGCTGTG CAACGTGGGC AAGTCATTGT CCTCTGAGCT GCAGTTTCCT 3000
CATCTGTCAC ATCGCTACAG ACAAGACCTC CCTGGAACCC TTCTGATTGT CTTAGACACT 3060
GTGGTTGCAA AACCCACGGA AAGCCTCATT TGTGTGGAAA GTCAGAGGAA AAATGATCCA 3120
GTGGACACTT GGGGATTATC TGTCATTCAA GATCCTTCCT TCAACCCCAA GGCCAGCTCC 3180

CATCTCATTT CCAGAAAGGC TCATACCTGG CTTGCAGGGA AGCATCTGTC TTGTCATTCC 3240
AGGTGCCAGA ATCCTCTCAG AGTCATTGAA GGGTGTTCAC CCATCCCACC CAAGGCTTGG 3300
CACACTGCCA GTGTCTTAGC AGGGTCTTGT GAGGGCTGGG GGCATCCAGG CACTCAGAAG 3360
GCAAAGGAAC CACCCTACCC ATTTGGCCTC TGGAGGGGGC AGAAGAAAGA AAGAAACCTC 3420
ATCCTATATT TTACAAAGCA TGTGAATTCT GGCATTAGCT CTCATAGGAG ACCCATGTGC 3480
TTCCTTGCTC AGTGCAAAAC TGATGATTCT ACTTGCTGTA GATGAATGGT TAACACGAGC 3540
TAGTTAAACA GTGCCATTGT TTTGCCAGTG AAGCCTCCAA CCCTAAGCCA CTGGGACGGT 3600
GGCCAGAGAT GCCAGCAGCC TCTGTCGCCC TTAGTCATAT AACCAAAATC CAGACCTTAT 3660
CCACAACCCG GGGCTTGGAA AGGAAGGTAT TTTGGAATCA CACCCTCCGG TTATGTTGCT 3720
CCAGTAAAAT CTTGCCTGGA AAGAGGCAGT CTTCTTAGCA TGGTGAGCTG AGTTCATGGC 3780
TTTTTTTTGT AGCCAGTCCT GTCCCTGGCC ATCCATGTGA TGGTTTTGGA TGGAGTTAAA 3840
CTTGATGCCA GTGGGCAGTG CATGTGGAAA GTATCAGAGT AAGCCTCTCC CCTCCAGAGC 3900
CCTGAGTTTC TTGGCTGCAT GAAGGTTTTC TTTAGAATCA GAATTGTAGC CAGTTTCTTT 3960
GGCCAGAAGG ATGAATACTT GGATATTACT GAAAGGGAGG GGTGGAGATG GGTGTGGCAG 4020
TGTATGGTGT GTGATTTTTA TTTTCTTCTT TGGTCATGGG GGCCAAGGAG AAAGGCATGA 4080
ATCTTCCCTG TCAGGCTCTT ACAGCCACAG GCACTGTGTC TACTGTCTGG AAGACATGTC 4140
CCCGTGGCTG TGGGGCCGCT GCTTCTGTTT AAATAAAAGT GGCCTGG 4187

## Description

Neurological diseases are widely spread within a population and have a strong impact not only on patients' life but also on society as such. Therefore, there is a strong need to elucidate the causes and the underlying pathogenesis of such neurological diseases. Among such neurological diseases, Alzheimer's disease (AD) has a predominant position. Alzheimer's disease, first described by the Bavarian psychiatrist Alois Alzheimer in 1907, is a progressive neurological disorder which begins with short term memory loss and proceeds to loss of cognitive functions, disorientation, impairment of judgment and reasoning and, ultimately, dementia. It is the most common cause of dementia. The neuropathology is characterized by the formation in brain of amyloid plaques and neurofibrillary tangles. AD has been estimated to afflict 5 to 11 percent of the population over age 65 and as much as 47 percent of the population over age 85. Moreover, as adults, born during the population boom of the 1940's and 1950's, approach the age when AD becomes more prevalent, the control and treatment of AD will become an even more significant health care problem. Familial forms of AD are genetically heterogeneous, but most with early onset are linked to mutations in the presenilin genes *PSEN1* and *PSEN2*, as well as to mutations of the amyloid precursor gene *APP*. The majority of AD patients have no obvious family history and are classified as sporadic AD.

As neurological diseases, in particular AD, are a growing social and medical problem, there is a strong need for methods of diagnosing or prognosing said diseases in subjects as well as for methods of treatment.

In one aspect, the invention features a protein molecule (hereinafter "SELADIN-1") shown in SEQ ID NO. 1.

In another aspect, the invention features an isolated nucleic acid molecule encoding SELADIN-1 shown in SEQ ID NO. 1. Said nucleic acid molecule can be a DNA molecule, such as a genomic DNA molecule or a cDNA molecule, or an RNA molecule, such as a mRNA molecule. In particular, said nucleic acid molecule can be a cDNA molecule having a coding sequence substantially the same as the coding sequence of SEQ ID NO. 2.

In another aspect, the invention features an oligonucleotide which hybridizes with a base sequence encoding SELADIN-1 shown in SEQ ID NO.1 in a solution of 0.2xSSC (standard saline citrate) and 0.1 % SDS at 60°C.

In another aspect, the invention features an isolated DNA comprising a base sequence which hybridizes with said base sequence encoding SELADIN-1 shown in SEQ ID NO. 1 in a solution of 50 % formamide, 4xSSC, 50 mM HEPES, pH 7.0, 10x Denhardt's solution, 100 µg/ml thermally denatured salmon sperm DNA at 42 °C. The invention further aims at an oligonucleotide which hybridizes with a base sequence comprising the afore mentioned DNA molecule in a solution of 0.2xSSC and 0.1 % SDS at 60 °C.

"Variants" of a protein molecule shown in SEQ ID NO.1 include e.g. proteins with conservative amino acid substitutions in highly conservative regions. For example, isoleucine, valine and leucine can each be substituted for one another. Aspartate and glutamate can be substituted for each other. Glutamine and asparagine can be substituted for each other. Serine and threonine can be substituted for each other. Amino acid substitutions in less conservative regions include e.g.: Isoleucine, valine and leucine can each be substituted for one another. Aspartate and glutamate can be substituted for each other. Glutamine and asparagine can be subsituted for each other. Serine and threonine can be substituted for each other. Glycine and alanine can be substituted for each other. Alanine and valine can be substituted for each other. Methionine can be substituted for each of leucine, isoleucine or valine, and vice versa. Lysine and arginine can be substituted for each other. One of aspartate and glutamate can be substituted for one of arginine or lysine, and vice versa. Histidine can be substituted for arginine or lysine, and vice versa. Glutamine and glutamate can be substituted for each other. Asparagine and aspartate can be substituted for each other.

"Variants" of SELADIN-1 shown in SEQ ID NO.1 also include e.g. protein molecules which are encoded by DNA fragments comprising base sequences which hybridize with said base sequence encoding SELADIN-1 shown in SEQ ID NO. 1 in a solution of 50 % formamide, 4xSSC, 50 mM HEPES, pH 7.0, 10x Denhard's solution, 100 µg/ml thermally denatured salmon sperm DNA at 42 °C.

The invention further features an antibody specifically immunoreactive with an immunogen, wherein said immunogen is SELADIN-1 shown in SEQ ID NO. 1 or a variant or fragment thereof. In another aspect, the invention aims at a method of detecting pathological structures in the brain of a subject which comprises immunocytochemically staining said pathological structures with said antibody.

In another aspect, the invention features a vector comprising the isolated nucleic acid encoding a SELADIN-1 shown in SEQ ID NO. 1 or a variant thereof. In preferred embodiments, a virus, a bacteriophage, or a plasmid comprises said nucleic acid.

In still another aspect, the invention features a plasmid adapted for expression in a bacterial cell which comprises a nucleic acid molecule encoding SELADIN-1 shown in SEQ ID NO. 1 or a variant thereof, and the regulatory elements necessary for expression of said molecule in the bacterial cell.

In a further aspect, the invention features a plasmid adapted for expression in a yeast cell which comprises a nucleic acid molecule encoding SELADIN-1 shown in SEQ ID NO. 1 or a variant thereof, and the regulatory elements necessary for expression of said molecule in the yeast cell.

In another aspect, the invention features a plasmid adapted for expression in a mammalian cell which comprises a nucleic acid molecule encoding SELADIN-1 shown in SEQ ID NO.1 or a variant thereof, and the regulatory elements necessary for expression of said molecule in the mammalian cell.

In a further aspect, the invention features a cell transformed with a nucleic acid molecule encoding SELADIN-1 shown in SEQ ID NO. 1 or a variant thereof. In preferred embodiments, said cell is a bacterial cell, a yeast cell, a mammalian cell, or an insect cell.

In a further aspect, the invention features a bacterial cell comprising a plasmid adapted for expression in a bacterial cell, said plasmid comprises a nucleic acid molecule encoding a protein molecule shown in SEQ ID NO. 1, and the regulatory elements necessary for expression of said molecule in the bacterial cell.

In a further aspect, the invention features a yeast cell comprising a plasmid adapted for expression in a yeast cell, said plasmid comprises a nucleic acid molecule encoding a protein molecule shown in SEQ ID NO. 1, and the regulatory elements necessary for expression of said molecule in the yeast cell.

In another aspect, the invention features a mammalian cell comprising a plasmid adapted for expression in a mammalian cell, said plasmid comprises a nucleic acid molecule encoding a protein molecule shown in SEQ ID NO.1, and the regulatory elements necessary for expression of said molecule in the mammalian cell.

In preferred embodiments, said subjects suffer from Alzheimer's disease and related neurofibrillary disorders, or neurodegenerative states characterized by cell degeneration or cell death. Further examples of neurological diseases are Parkinson's disease, Huntington disease, Amyotrophic lateralsclerosis, Pick's disease.

In another aspect, the invention features a method of diagnosing or prognosing a neurological disease in a subject comprising:
determining a level, or an activity, or both said level and said activity, of at least one substance which is selected from the group consisting of
   a) a protein molecule shown in SEQ ID NO. 1,
   b) a variant or fragment thereof,
   c) a nucleic acid molecule encoding a protein molecule shown in SEQ ID NO. 1, a variant or fragment thereof,
   d) a molecule affecting directly or indirectly a level, or an activity, or both said level and said activity, of at least one substance which is selected from the group consisting of a) to c),
   e) a molecule which is affected in its level, or its activity, or both its level and activity, by at least one substance which is selected from the group consisting of a) to c),
in a sample from said subject,
   and
comparing said level, or said activity, or both said level and said activity, of at least one of said substances a) to e) to a reference value representing a known disease or health status,
thereby diagnosing or prognosing said neurological disease in said subject.

In preferred embodiments, said subjects suffer from Alzheimer's disease and related neurofibrillary disorders, or neurodegenerative states characterized by cell degeneration or cell death. Further examples of neurological diseases are Parkinson's disease, Huntington disease, Amyotrophic lateralsclerosis, Pick's disease.

It is particularly preferred that said sample is a brain tissue or other body cells including blood cells and skin fibroblasts. According to the present invention, a reduction in the level, or activity, or both said level and said activity, of SELADIN-1 or *SELADIN-1* transcripts in said subject's brain regions affected heavily by neurodegeneration relative to a reference value indicates a diagnosis or prognosis of Alzheimer's disease. Predominantly neurons within the inferior temporal lobe, the entorhinal cortex, the hippocampus and the amygdala degenerate in Alzheimer's disease.

It is also particularly preferred that said sample is cerebrospinal fluid or another body fluid.

It might be preferred that said subject has previously been determined to have one or more factors indicating that such subject is afflicted with a neurological disease.

In preferred embodiments, said subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In preferred embodiments, at least one of said substances is detected using an immunoassay, an enzyme activity assay and/or a binding assay.

In preferred embodiments, measurement of the level of transcription products of the *SELADIN-1* gene, or a variant thereof, is performed in body cells using Northern blots with probes specific for the *SELADIN-1* gene or said variant. Quantitative PCR with primer combinations to amplify *SELADIN-1* gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed. W.H. Freeman and Company, 1992).

In preferred embodiments, said level or activity of the protein molecule shown in SEQ ID NO. 1, or a variant or fragment thereof, is detected using an immunoassay. These assays can measure the amount of binding between said protein molecule ("SELADIN-1") and an anti-SELADIN-1 antibody by the use of enzymatic, chromodynamic, radioactive, or luminescent labels which are attached to either the anti-SELADIN-1 antibody or a secondary antibody which binds the anti-SELADIN-1 antibody. In addition, other high affinity ligands may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect SELADIN-1 or a variant or fragment thereof. It is preferred that it does not substantially interact with any other protein present in said sample.

Monoclonal antibodies capable of recognizing a protein molecule of SEQ ID NO. 1 or a variant or fragment thereof can be prepared using methods known in the art (see e.g. Köhler and Milstein, Nature 256, 495 - 497 1975; Kozbor at al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; ; Watson et al., Rekombinierte DNA, 2nd ed., Spektrum Akademischer Verlag GmbH, 1993; Watson et al, Recombinant DNA, 2nd ed., W. H. Freeman and Company, 1992; the contents of which are incorporated herein by reference). Monoclonal antibodies useful in the methods of the invention are directed to an epitope of SELADIN-1 or a variant or fragment thereof, such that the complex formed between the antibody and SELADIN-1, or between the antibody and said variant or fragment, can be recognized in detection assays. The term "antibodies" encompasses all forms of antibodies known in the art, such as polyclonal, monoclonal, chimeric, recombinatorial, single chain antibodies as well as fragments thereof which specifically bind to SELADIN-1, or to a variant or fragment thereof.

Antibodies or ligands might also be used in detecting specifically molecules mentioned under d) and e) above.

If luminescent labels are used in any detection assay, it is preferred to use a confocal optical set-up.

In further preferred embodiments, said reference value is that of a level, or an activity, or both said level and said activity, of at least one substance which is selected from the group consisting of a) to e) in a sample from a subject not suffering of said neurological disease. The healthy subject can be of the same weight, age, and gender as the subject who is being diagnosed or prognosed for said neurological disease. In some cases, it might be preferred to use a reference value from the subject which is diagnosed.

In another aspect, the invention features a method of monitoring the progression of a neurological disease in a subject, comprising:
determining a level, or an activity, or both said level and said activity, of at least one substance which is selected from the group consisting of
   a) a protein molecule shown in SEQ ID NO. 1,
   b) a variant or fragment thereof,
   c) a nucleic acid molecule encoding a protein molecule shown in SEQ ID NO. 1, a variant or fragment thereof,
   d) a molecule affecting directly or indirectly a level, or an activity, or both said level and said activity, of at least one substance which is selected from the group consisting of a) to c),
   e) a molecule which is affected in its level, or its activity, or both its level and activity, by at least one substance which is selected from the group consisting of a) to c),
in a sample from said subject;
   and
comparing said level, or said activity, or both said level and said activity, of at least one of said substances a) to e) to a reference value representing a known disease or health status,
thereby monitoring the progression of said neurological disease in said subject.

In a preferred embodiment, the level, or the activity, or both said level and said activity, of at least one of said substances a) to e) in a sample is determined at least twice, e.g. at two points which are weeks or months apart. The levels or activities at these two time points are compared in order to monitor the progression of said neurological disease. It might be preferred to take a series of samples over a period of time. In further preferred embodiments, said subject receives a treatment prior to one or more of said sample gatherings.

In preferred embodiments, said subjects suffer from Alzheimer's disease and related neurofibrillary disorders, or neurodegenerative states characterized by cell degeneration or cell death. Further examples of neurological diseases are Parkinson's disease, Huntington disease, Amyotrophic lateralsclerosis, Pick's disease.

It is particularly preferred that said sample is a brain tissue or other body cells including blood cells and skin fibroblasts. According to the present invention, a reduction in the level, or activity, or both said level and said activity, of SELADIN-1 or *SELADIN-1* transcripts in said subject's brain regions affected heavily by neurodegeneration relative to a reference value indicates a diagnosis or prognosis of Alzheimer's disease. Predominantly neurons within the inferior temporal lobe, the entorhinal cortex, the hippocampus and the amygdala degenerate in Alzheimer's disease.

It is also particularly preferred that said sample is cerebrospinal fluid or another body fluid.

It might be preferred that said subject has previously been determined to have one or more factors indicating that such subject is afflicted with a neurological disease.

In preferred embodiments, said subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In preferred embodiments, at least one of said substances is detected using an immunoassay, an enzyme activity assay and/or a binding assay.

In preferred embodiments, measurement of the level of transcription products of the *SELADIN-1* gene, or a variant thereof, is performed in body cells using Northern blots with probes specific for the *SELADIN-1* gene or said variant. Quantitative PCR with primer combinations to amplify *SELADIN-1* gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed. W.H. Freeman and Company, 1992).

In preferred embodiments, said level or activity of the protein molecule shown in SEQ ID NO. 1, or a variant or fragment thereof, is detected using an immunoassay. These assays can measure the amount of binding between said protein molecule ("SELADIN-1") and an anti-SELADIN-1 antibody by the use of enzymatic, chromodynamic, radioactive, or luminescent labels which are attached to either the anti-SELADIN-1 antibody or a secondary antibody which binds the anti-SELADIN-1 antibody. In addition, other high affinity ligands may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect SELADIN-1 or a variant or fragment thereof. It is preferred that it does not substantially interact with any other protein present in said sample.

Monoclonal antibodies capable of recognizing a protein molecule of SEQ ID NO. 1 or a variant or fragment thereof can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; ; Watson et al., Rekombinierte DNA, 2nd ed., Spektrum Akademischer Verlag GmbH, 1993; Watson et al, Recombinant DNA, 2nd ed., W. H. Freeman and Company, 1992; the contents of which are incorporated herein by reference). Monoclonal antibodies useful in the methods of the invention are directed to an epitope of SELADIN-1 or a variant or fragment thereof, such that the complex formed between the antibody and SELADIN-1, or between the antibody and said variant or fragment, can be recognized in detection assays. The term "antibodies" encompasses all forms of antibodies known in the art, such as polyclonal, monoclonal, chimeric, recombinatorial, single chain antibodies as well as fragments thereof which specifically bind to SELADIN-1, or to a variant or fragment thereof.

Antibodies or ligands might also be used in detecting specifically any molecules mentioned under d) to e) above.

If luminescent labels are used in any detection assay, it is preferred to use a confocal optical set-up.

In further preferred embodiments, said reference value is that of a level, or an activity, or both said level and said activity, of at least one substance which is selected from the group consisting of a) to e) in a sample from a subject not suffering of said neurological disease. The healthy subject can be of the same weight, age, and gender as the subject who is being diagnosed or prognosed for said neurological disease. In some cases, it might be preferred to use a reference value from the subject which is diagnosed.

In still another aspect, the invention features a method of evaluating a treatment for a neurological disease, comprising:
determining a level, or an activity, or both said level and said activity, of at least one substance which is selected from the group consisting of
   a) a protein molecule shown in SEQ ID NO. 1,
   b) a variant or fragment thereof,
   c) a nucleic acid molecule encoding a protein molecule shown in SEQ ID NO. 1, a variant or fragment thereof,
   d) a molecule affecting directly or indirectly a level, or an activity, or both said level and said activity, of at least one substance which is selected from the group consisting of a) to c),
   e) a molecule which is affected in its level, or its activity, or both its level and activity, by at least one substance which is selected from the group consisting of a) to c),
in a sample from said subject;
   and
comparing said level, or said activity, or both said level and said activity, of at least one of said substances a) to e) to a reference value representing a known disease or health status,
thereby evaluating said treatment for said neurological disease.

In preferred embodiments, said subjects suffer from Alzheimer's disease and related neurofibrillary disorders, or neurodegenerative states characterized by cell degeneration or cell death. Further examples of neurological diseases are Parkinson's disease, Huntington disease, Amyotrophic lateralsclerosis, Pick's disease.

It is particularly preferred that said sample is a brain tissue or other body cells including blood cells and skin fibroblasts. According to the present invention, a reduction in the level, or activity, or both said level and said activity, of SELADIN-1 or *SELADIN-1* transcripts in said subject's brain regions affected heavily by neurodegeneration relative to a reference value indicates a diagnosis or prognosis of Alzheimer's disease. Predominantly neurons within the inferior temporal lobe, the entorhinal cortex, the hippocampus and the amygdala degenerate in Alzheimer's disease.

It is also particularly preferred that said sample is cerebrospinal fluid or another body fluid.

It might be preferred that said subject has previously been determined to have one or more factors indicating that such subject is afflicted with a neurological disease.

In preferred embodiments, said subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In preferred embodiments, at least one of said substances is detected using an immunoassay, an enzyme activity assay and/or a binding assay.

In preferred embodiments, measurement of the level of transcription products of the *SELADIN-1* gene, or a variant thereof, is performed in body cells using Northern blots with probes specific for the *SELADIN-1* gene or said variant. Quantitative PCR with primer combinations to amplify *SELADIN-1* gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed. W.H. Freeman and Company, 1992).

In preferred embodiments, said level or activity of the protein molecule shown in SEQ ID NO. 1, or a variant or fragment thereof, is detected using an immunoassay. These assays can measure the amount of binding between said protein molecule ("SELADIN-1") and an anti-SELADIN-1 antibody by the use of enzymatic, chromodynamic, radioactive, or luminescent labels which are attached to either the anti-SELADIN-1 antibody or a secondary antibody which binds the anti-SELADIN-1 antibody. In addition, other high affinity ligands may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect SELADIN-1 or a variant or fragment thereof. It is preferred that it does not substantially interact with any other protein present in said sample.

Monoclonal antibodies capable of recognizing a protein molecule of SEQ ID NO. 1 or a variant or fragment thereof can be prepared using methods known in the art (see e.g. Köhler and Milstein, Nature 256, 495 - 497 1975; Kozbor et al., Immunol. Today 4, 72, 1983; Cole at al., Monoclonal antibodies and cancer therapy, Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; ; Watson et al., Rekombinierte DNA, 2nd ed., Spektrum Akademischer Verlag GmbH, 1993; Watson et al, Recombinant DNA, 2nd ed., W. H. Freeman and Company, 1992; the contents of which are incorporated herein by reference). Monoclonal antibodies useful in the methods of the invention are directed to an epitope of SELADIN-1 or a variant or fragment thereof, such that the complex formed between the antibody and SELADIN-1, or between the antibody and said variant or fragment, can be recognized in detection assays. The term "antibodies" encompasses all forms of antibodies known in the art, such as polyclonal, monoclonal, chimeric, recombinatorial, single chain antibodies as well as fragments thereof which specifically bind to SELADIN-1, or to a variant or fragment thereof.

Antibodies or ligands might also be used in detecting specifically any molecules mentioned under d) to e) above.

If luminescent labels are used in any detection assay, it is preferred to use a confocal optical set-up.

In further preferred embodiments, said reference value is that of a level, or an activity, or both said level and said activity, of at least one substance which is selected from the group consisting of a) to e) in a sample from a subject not suffering of said neurological disease. The healthy subject can be of the same weight, age, and gender as the subject who is being diagnosed or prognosed for said neurological disease. In some cases, it might be preferred to use a reference value from the subject which is diagnosed.

In a further aspect, the invention features a kit for diagnosis, or prognosis, of a neurological disease, said kit comprising:
(1) at least one reagent which is selected from the group consisting of reagents that selectively detect
   a) a protein molecule shown in SEQ ID NO.1,
   b) a variant or fragment thereof,
   c) a nucleic acid molecule encoding a protein molecule shown in SEQ ID NO.1, a variant or fragment thereof,
   d) a molecule affecting directly or indirectly a level, or an activity, or both said level and said activity, of at least one substance which is selected from the group consisting of a) to c),
   e) a molecule which is affected in its level, or its activity, or both its level and activity, by at least one substance which is selected from the group consisting of a) to c), and
(2) instructions for diagnosing, or prognosing said neurological disease by
   (i) detecting a level, or an activity, or both said level and said activity, of at least one substance which is selected from the group consisting of a) to e)
      in a sample from said subject;
      and
   (ii) diagnosing, or prognosing said neurological disease, wherein
      a varied level, or varied activity, or both said varied level and said varied activity, of at least one substance which is selected from the group consisting of a) to e) compared to a reference value representing a known health status;
      or a level, or an activity, or both said level and said activity, of at least one substance which is selected from the group consisting of a) to e) similar or equal to a reference value representing a known disease status indicates diagnosis, or prognosis of said neurological disease.

In preferred embodiments, said subjects suffer from Alzheimer's disease and related neurofibrillary disorders, or neurodegenerative states characterized by cell degeneration or cell death. Further examples of neurological diseases are Parkinson's disease, Huntington disease, Amyotrophic lateralsclerosis, Pick's disease.

It is particularly preferred that said sample is a brain tissue or other body cells including blood cells and skin fibroblasts. According to the present invention, a reduction in the level, or activity, or both said level and said activity, of *SELADIN-1* transcripts in said subject's brain regions affected heavily by neurodegeneration relative to a reference value indicates a diagnosis or prognosis of Alzheimer's disease. Predominantly neurons within the inferior temporal lobe, the entorhinal cortex, the hippocampus and the amygdala degenerate in Alzheimer's disease.

It is also particularly preferred that said sample is cerebrospinal fluid or another body fluid.

It might be preferred that said subject has previously been determined to have one or more factors indicating that such subject is afflicted with a neurological disease.

In preferred embodiments, said subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In preferred embodiments, at least one of said substances a) to e) is detected using an immunoassay, an enzyme activity assay and/or a binding assay.

In preferred embodiments, measurement of the level of transcription products of the *SELADIN-1* gene, or a variant thereof, is performed in body cells using Northern blots with probes specific for the *SELADIN-1* gene or said variant. Quantitative PCR with primer combinations to amplify *SELADIN-1* gene-specific sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed. W.H. Freeman and Company, 1992).

In preferred embodiments, said level or activity of the protein molecule shown in SEQ ID NO. 1, or a variant or fragment thereof, is detected using an immunoassay. These assays can measure the amount of binding between said protein molecule ("SELADIN-1") and an anti-SELADIN-1 antibody by the use of enzymatic, chromodynamic, radioactive, or luminescent labels which are attached to either the anti-SELADIN-1 antibody or a secondary antibody which binds the anti-SELADIN-1 antibody. In addition, other high affinity ligands may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect SELADIN-1 or a variant or fragment thereof. It is preferred that it does not substantially interact with any other protein present in said sample.

Monoclonal antibodies capable of recognizing a protein molecule of SEQ ID NO. 1 or a variant or fragment thereof can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy. Alan R. Liss, Inc., pp 77 - 96, 1985; Marks et al., J. Biol. Chem., 16007 - 16010, 1992; the contents of which are incorporated herein by reference). Such monoclonal antibodies or fragments thereof can also be produced by alternative methods known to those of skill in the art of recombinant DNA technology (see e.g. Sastry et al, PNAS 86: 5728, 1989; ; Watson et al., Rekombinierte DNA, 2nd ed., Spektrum Akademischer Verlag GmbH, 1993; Watson et al, Recombinant DNA, 2nd ed., W. H. Freeman and Company, 1992; the contents of which are incorporated herein by reference). Monoclonal antibodies useful in the methods of the invention are directed to an epitope of SELADIN-1 or a variant or fragment thereof, such that the complex formed between the antibody and SELADIN-1, or between the antibody and said variant or fragment, can be recognized in detection assays. The term "antibodies" encompasses all forms of antibodies known in the art, such as polyclonal, monoclonal, chimeric, recombinatorial, single chain antibodies as well as fragments thereof which specifically bind to SELADIN-1, or to a variant or fragment thereof.

Antibodies or ligands might also be used in detecting specifically any molecules mentioned under d) to e) above.

If luminescent labels are used in any detection assay, it is preferred to use a confocal optical set-up.

In further preferred embodiments, said reference value is that of a level, or an activity, or both said level and said activity, of at least one substance which is selected from the group consisting of a) to e) in a sample from a subject not suffering of said neurological disease. The healthy subject can be of the same weight, age, and gender as the subject who is being diagnosed or prognosed for said neurological disease. In some cases, it might be preferred to use a reference value from the subject which is diagnosed.

In another aspect, the kit may be used in monitoring a progression of said neurological disease in a subject.

In a further aspect, the kit may be used in monitoring success or failure of a therapeutic treatment of said subject.

In another aspect, the invention features a method of treating or preventing a neurological disease in a subject comprising administering to said subject in a therapeutically effective amount an agent or agents which directly or indirectly affect a level, or an activity, or both said level and said activity, of at least one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and said activity, of at least one substance which is selected from the group consisting of a) to c),
e) a molecule which is affected in its level, or its activity, or both its level and activity, by at least one substance which is selected from the group consisting of a) to c).

In preferred embodiments, the method comprises the application of per se known methods of gene therapy and/or antisense nucleic acid technology to administer said agent or said agents.

In general, gene therapy includes several approaches: molecular replacement of a mutated gene, addition of a new gene resulting in the synthesis of a therapeutic protein, and modulation of endogeneous cellular gene expression by recombinant expression methods or by drugs. Gene-transfer techniques are described in detail (see e.g. Behr, Acc. Chem. Res. 26, 274 - 278, 1993; Mulligan, Science 260, 926 - 931, 1993; the contents of which are incorporated herein by reference) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane perturbation by chemical (solvents, detergents, polymers, enzymes) or physical means (mechanic, osmotic, thermic, electric shocks). The postnatal gene transfer into the central nervous system has been described in detail (see e.g. Wolff, Current Opinion in Neurobiology, 3, 743 - 748, 1993; the contents of which are incorporated herein by reference).

In particular, the invention features a method of treating or preventing a neurological disease, such as Alzheimer's disease, by means of antisense nucleic acid therapy, i.e. the down-regulation of an inappropriately expressed or defective gene by the introduction of antisense nucleic acids or derivatives thereof into certain critical cells (see e.g. Gillespie, DN & P 5(7), 389 - 395, 1992; Agrawal, Tibtech 13, 197 - 199, 1995; Crooke, Bio/Technology 10, 882 - 886, 1992; the contents of which are incorporated herein by reference). Apart from hybridization strategies, the application of ribozymes, i.e. RNA molecules that act as enzymes, destroying RNA that carries the message of disease has also been described (see e.g. Barinaga, Science, 262, 1512 - 1514, 1993; the contents of which are incorporated herein by reference). It is particularly preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligodeoxynucleotides are known to those of skill in the art (see e.g. Wickstrom, Tibtech, 10, 281 - 287, 1992; the contents of which are incorporated herein by reference). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of the target nucleic acid. Therapeutically use of intracellularly expressed antisense RNA is procedurally similar to gene therapy.

In preferred embodiments, the method comprises grafting donor cells into the central nervous system, preferably the brain, of said subject, said subject or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phospate transfection, liposomal mediated transfection, etc.

In preferred embodiments, said agent is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

In preferred embodiments, the therapeutic nucleic acid or protein reduces or prevents the degeneration of neurons and slowes brain amyloid formation.

In another aspect, the invention features an agent which directly or indirectly affects an activity, or level, or both said activity or level, of at least one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and said activity, of at least one substance which is selected from the group consisting of a) to c),
e) a molecule which is affected in its level, or its activity, or both its level and activity, by at least one substance which is selected from the group consisting of a) to c).

In another aspect, the invention features a medicament comprising such an agent.

In still another aspect, the invention features an agent for treating or preventing a neurological disease, which agent directly or indirectly affects an activity, or level, or both said activity or level, of at least one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and said activity, of at least one substance which is selected from the group consisting of a) to c),
e) a molecule which is affected in its level, or its activity, or both its level and activity, by at least one substance which is selected from the group consisting of a) to c).

In preferred embodiments, said neurological diseases are Alzheimer's disease and related neurofibrillary disorders, or neurodegenerative states characterized by cell degeneration or cell death. Further examples of neurological diseases are Parkinson's disease, Huntington disease, Amyotrophic lateralsclerosis, Pick's disease.

In a further aspect, the invention features the use of an agent, for treating or preventing a neurological disease, which agent directly or indirectly affects an activity, or level, or both said activity or level, of at (east one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and said activity, of at least one substance which is selected from the group consisting of a) to c),
e) a molecule which is affected in its level, or its activity, or both its level and activity, by at least one substance which is selected from the group consisting of a) to c).

In preferred embodiments, said neurological diseases are Alzheimer's disease and related neurofibrillary disorders, or neurodegenerative states characterized by cell degeneration or cell death. Further examples of neurological diseases are Parkinson's disease, Huntington disease, Amyotrophic lateralsclerosis, Pick's disease.

In a further aspect, the invention features a method for identifying an agent that directly or indirectly affects an activity, or level, or both said activity or level, of at least one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and said activity, of at least one substance which is selected from the group consisting of a) to c),
e) a molecule which is affected in its level, or its activity, or both its level and activity, by at least one substance which is selected from the group consisting of a) to c), comprising the steps of:
   (i) providing a sample containing at least one substance which is selected from the group consisting of a) to e);
   (ii) contacting said sample with at least one agent;
   (iii) comparing an activity, or level, or both said activity and level, of at least one of said substances before and after said contacting.

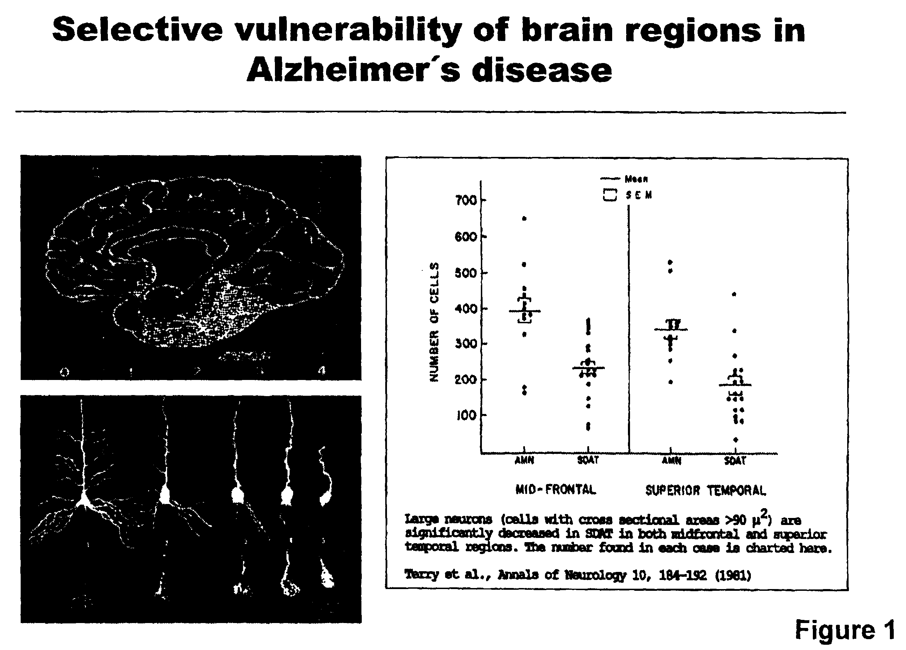

Figure 1 depicts the selective vulnerability of brain regions in Alzheimer's disease. Predominantly neurons within the inferior temporal lobe, the entorhinal cortex, the hippocampus and the amygdala degenerate in Alzheimer's disease (Terry et al., Annals of Neurology, 10, 184-192, 1981). These brain regions are predominantly involved in the processing of learning and memory functions. In contrast neurons within the frontal cortex, the occipital cortex and the cerebellum are largely intact and preserved from the neurodegenerative process in Alzheimer's disease.

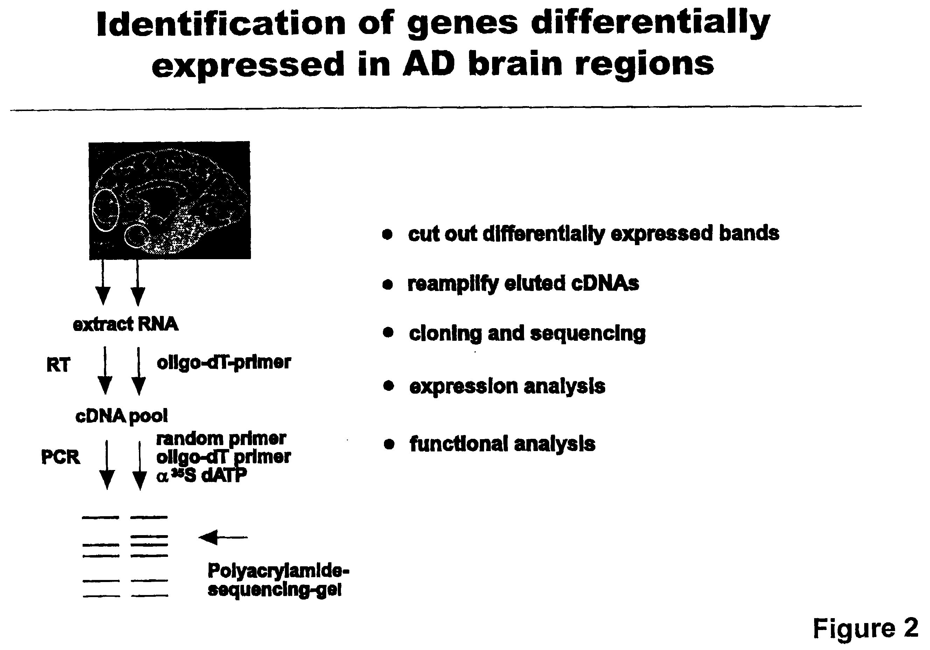

Figure 2 discloses the identification of genes differentially expressed in brain regions from Alzheimer's disease patients. Brain areas with massive neuronal cell loss as well as areas with largely preserved neurons were identified and RNA extracted. Synthesis of cDNA was performed using an oligo-dT primer followed by PCR using the oligo-dT primer in combination with random primers and (α³⁵S)-dATP. Reactions were separated on DNA sequencing gels, DNA bands visualized by autoradiography and bands lighting up in different intensities were cut out. DNA fragments were reamplified by PCR, cloned in *E. coli* and sequences determined. Expression and functional analyses were performed.

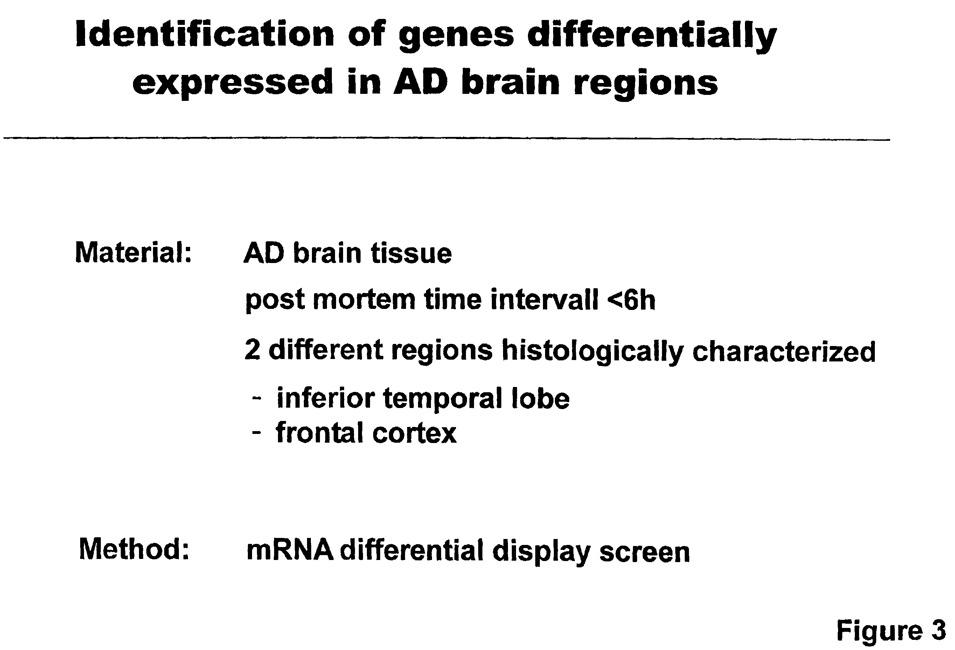

Figure 3 depicts the specifications of Alzheimer's disease brain tissue as it was used in the examples. Brain tissues from Alzheimer's disease patients and control subjects were removed within 6 hours of death, and immediately frozen on dry ice. For RNA extraction tissue sections from the inferior temporal lobe and frontal cortex were used.

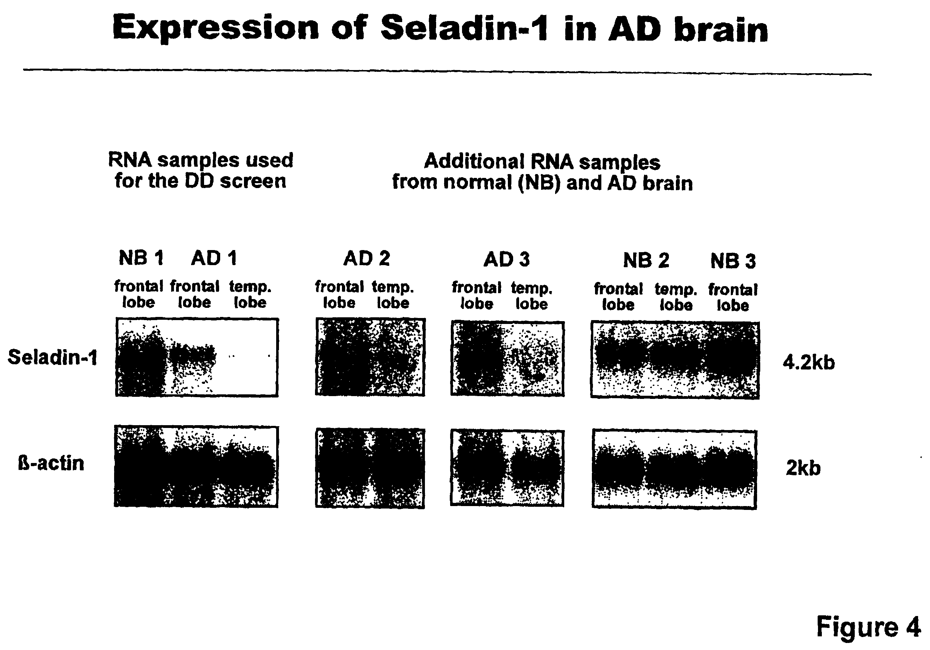

Figure 4 discloses the quantification of *SELADIN-1* transcripts in brain tissue from Alzheimer's disease and control subjects by Northern blot analyses. Transcript levels were significantly lower in brain regions with severe neurodegeneration, i. e. temporal lobe in Alzheimer's disease (AD1-3) but not in normal brain (NB1-3), as compared to protected brain regions, i. e. frontal lobe. This decrease was specific as indicated by unchanged β-actin transcript levels used to control for equal loading of RNA.

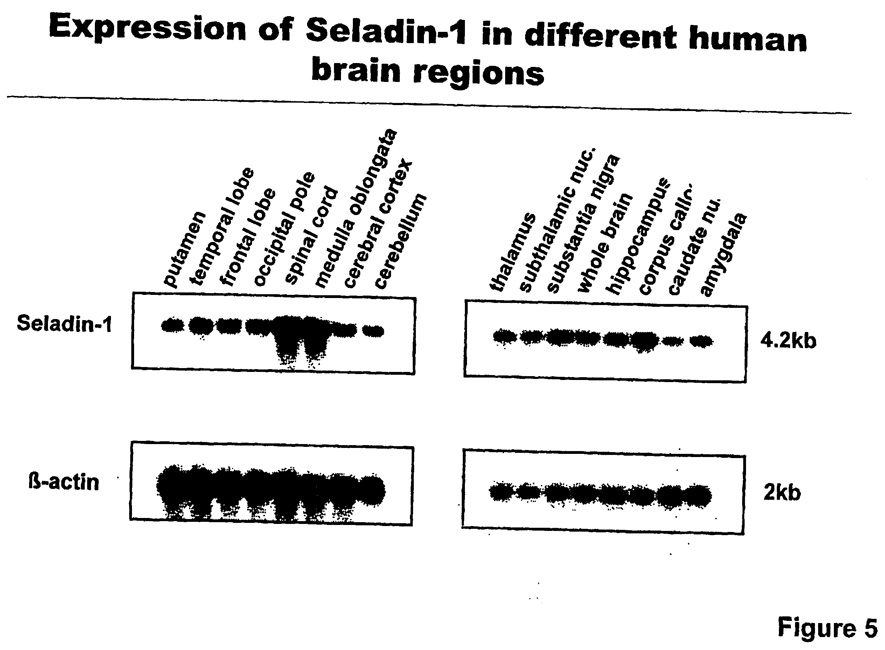

Figure 5 depicts the transcription levels of the *SELADIN-1* gene in different human brain regions. The *SELADIN-1* gene was found to be expressed throughout the human brain. In particular transcription levels are high in all cortical areas, the hippocampus, the amygdala, the spinal cord, and the medulla. Note the unchanged levels in temporal lobe versus frontal lobe in this brain derived from a cognitively normal control subject without any signs of Alzheimer's disease. The analysis of β-actin transcripts was used as loading control.

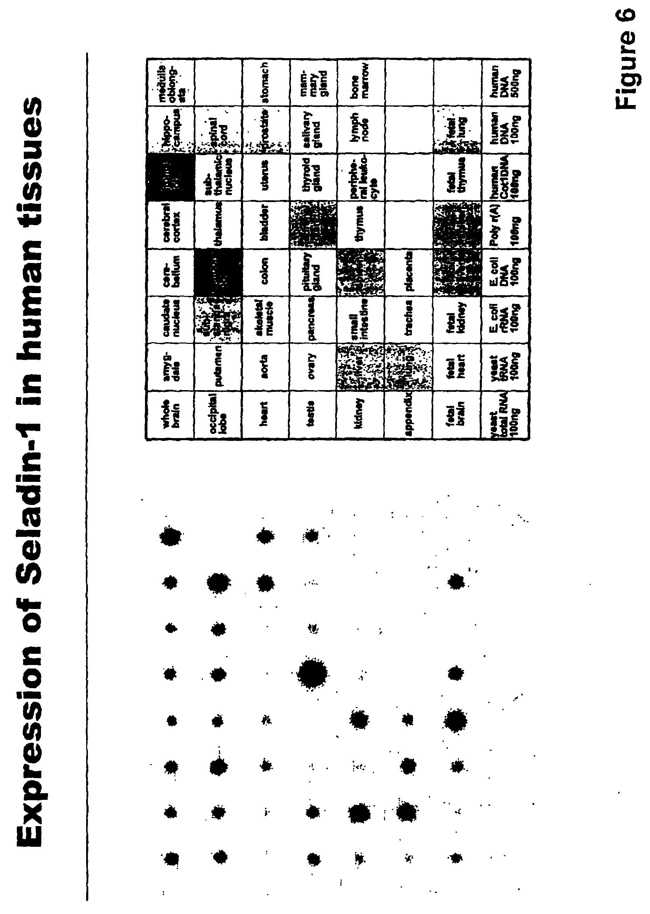

Figure 6 depicts the distribution of *SELADIN-1* transcripts in human tissues. Comparable samples of RNA were spotted on nitrocellulose filters and *SELADIN-1* transcripts were quantified by hybridization using a labeled SELADIN-1 gene specific probe. Significant levels of *SELADIN-1* gene transcripts were found in all brain regions tested. Transcripts were also detected in other tissues, however, strong variations in signal intensity indicated a tissue specific regulation of *SELADIN-1* expression.

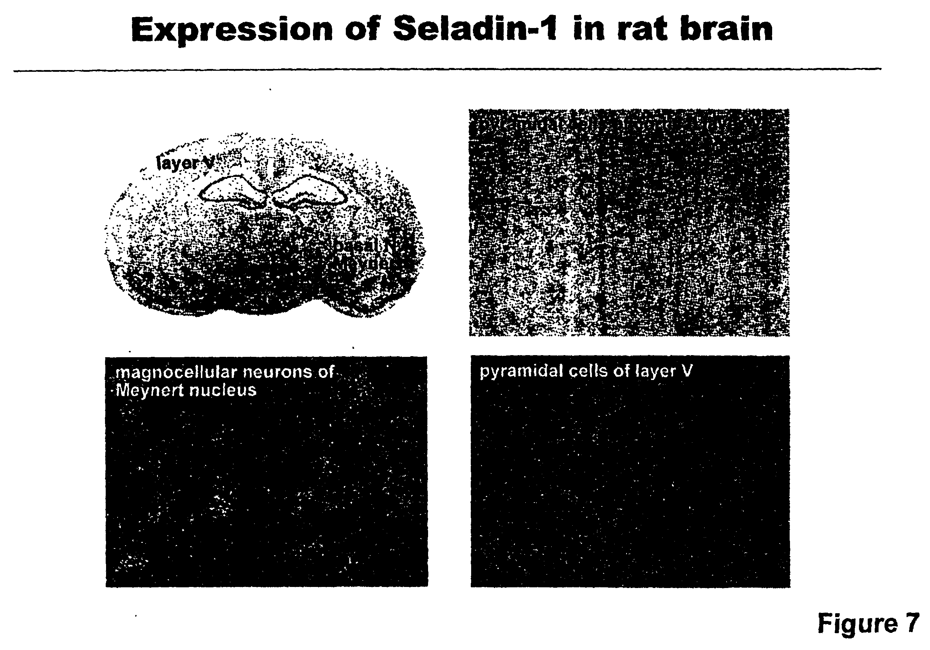

Figure 7 depicts the expression of the *SELADIN-1* gene in rat brain cortex, hippocampus and basal nucleus analyzed by in situ hybridization. This staining pattern along with the higher magnifications indicate that *SELADIN-1* is predominantly expressed in neurons. No significant hybridization signals were observed with glial cells.

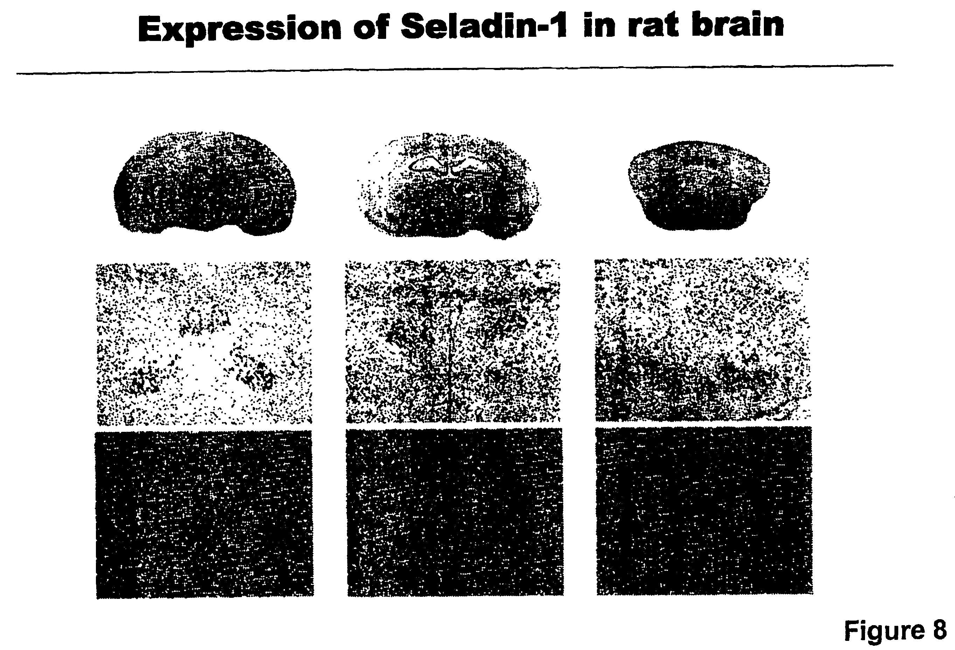

Figure 8 depicts the expression of *SELADIN-1* in rat brain nuclei. Strong *SELADIN-1* expression was found in the occulomotor, paraventricular, red and facial nuclei. Higher magnifications indicate predominant hybridization with neurons. No significant hybridization signals were observed with glial cells.

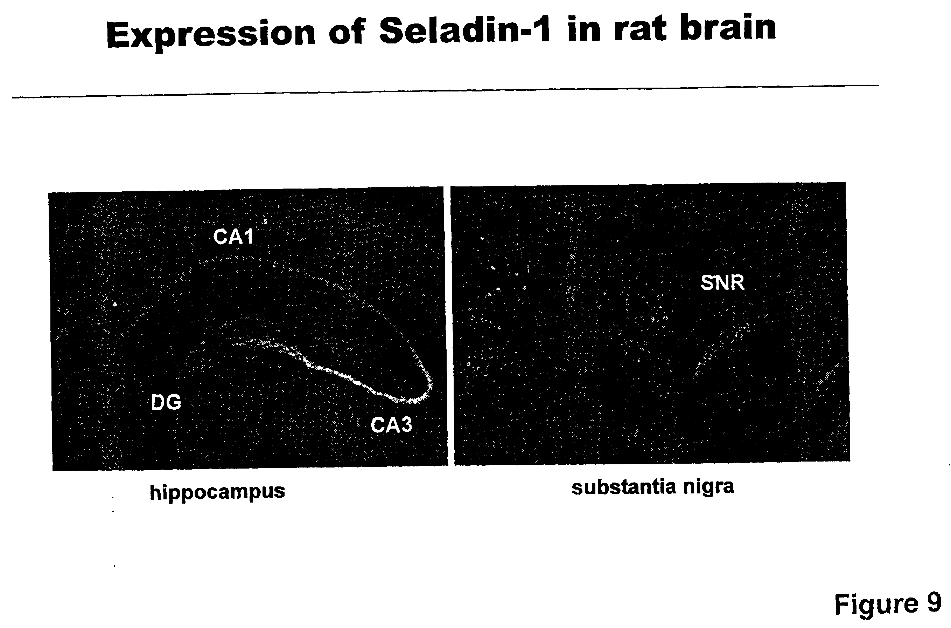

Figure 9 depicts the expression of *SELADIN-1* in rat brain hippocampus and substantia nigra. In situ hybridization with *SELADIN-1* transcripts was detected by photoemulsionautoradiography, confirming the neuron specific expression of this gene.

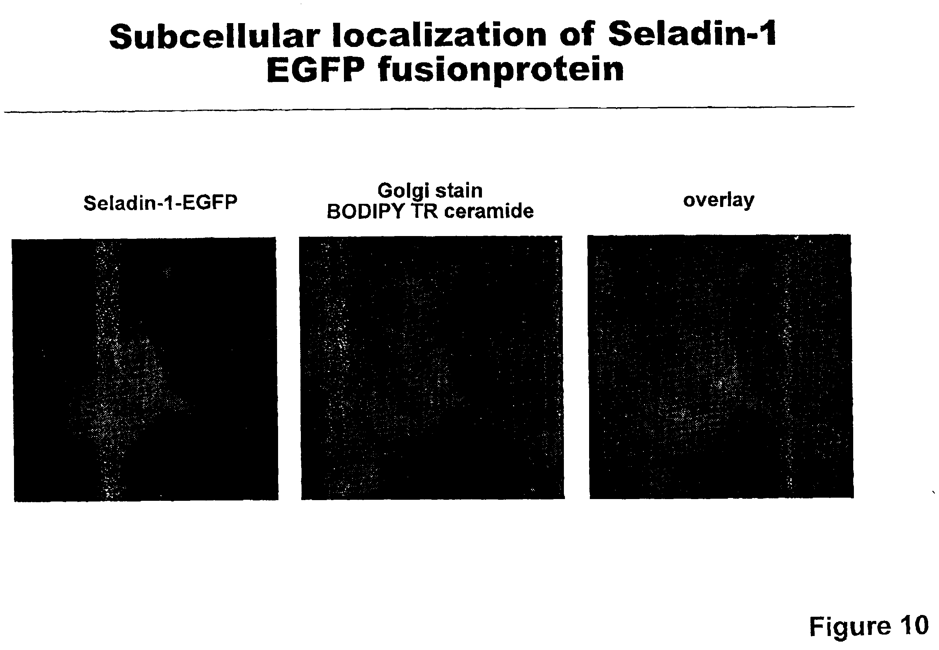

Figure 10 discloses the subcellular localization of a SELADIN-1-EGFP (enhanced green fluorescent protein) fusion in transfected Cos cells. The confocal micrographs show the co-localization of the SELADIN-1-EGFP fusion with the golgi specific stain BODIPY TR ceramide indicating localization of SELADIN-1 in the Golgi apparatus and the endoplasmic reticulum.

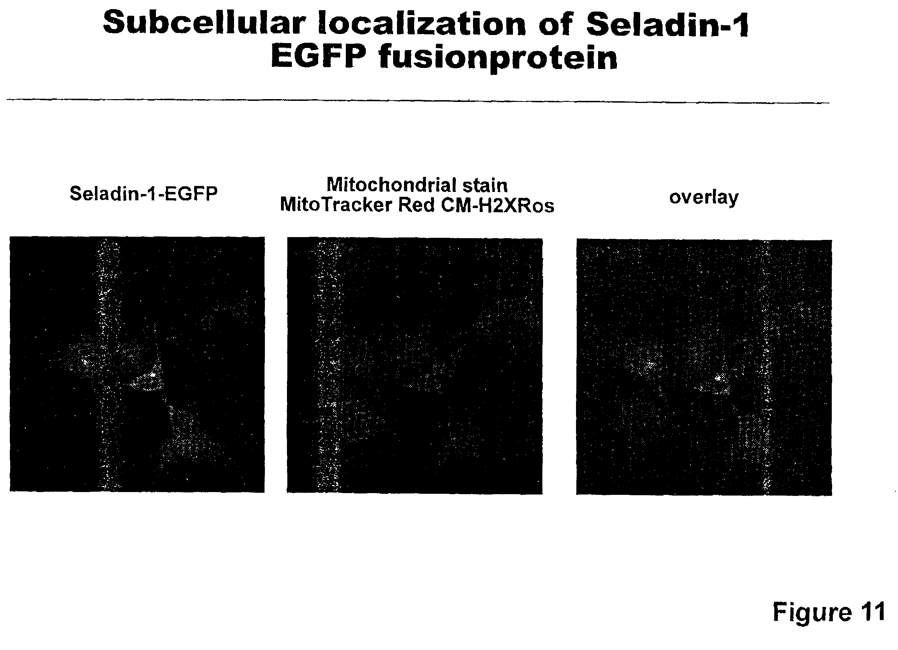

Figure 11 discloses that the SELADIN-1-EGFP fusion does not localize to mitochondria in transfected cos cells in spite of a putative mitochondrial targeting sequence close to the N-terminus of the SELADIN-1 protein. The confocal micrographs show the different staining patterns caused by the SELADIN-1-EGFP fusion and the specific mitochondrial stain Mito Tracker Red CM-H₂XRos.

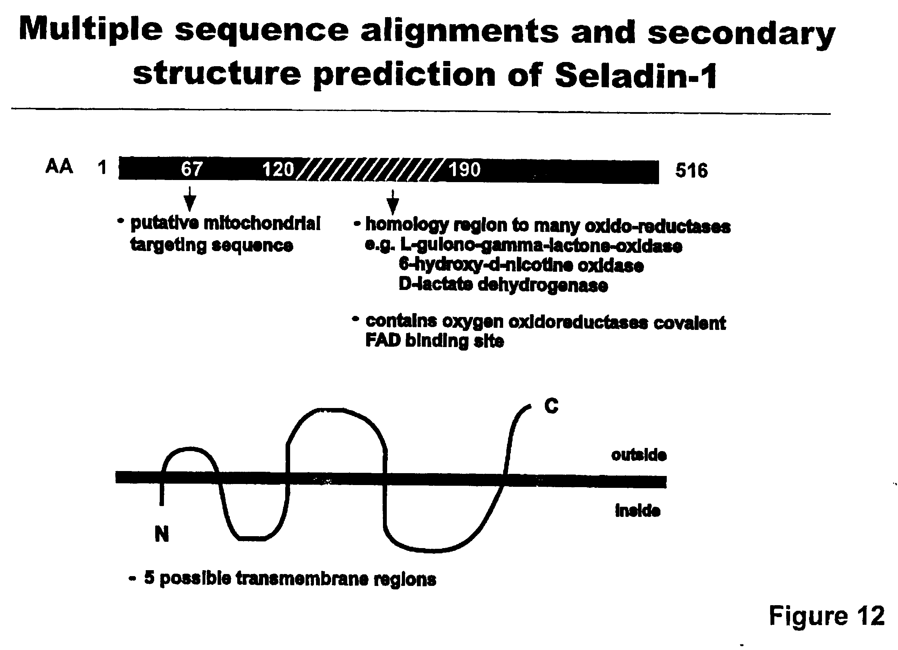

Figure 12 discloses structural features of the SELADIN-1 protein based on multiple sequence alignments and secondary structure predictions. Near the N-terminus the SELADIN-1 protein contains a putative mitochondrial localization signal that appears to be inactive in transfected Cos cells or when used in EGFP fusions. The central region of the protein contains a sequence that is homologous to a family of oxidoreductases and that contains a FAD site for covalent binding. The protein is predicted to contain five transmembrane regions. The expression in neurons, the co-localization in the Golgi apparatus and the endoplasmic reticulum of the SELADIN-1 protein, the amyloid precursor protein (APP) and the presenilins PS1 and PS2 and furthermore the transmembrane character suggest a functional relationship between these proteins. Mutations in both APP and presenilins were shown to cause an increase in the production of β-amyloid. In a similar way the SELADIN-1 protein might be involved in common biological pathways influencing the processing of the amyloid precursor protein and the generation of Aβ. Using the SELADIN-1 protein as a probe, interaction partners can be identified which might represent new AD drug targets.

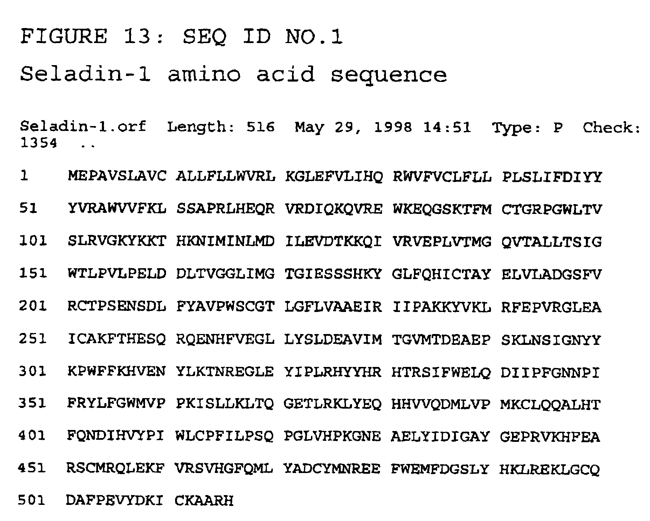

Figure 13 discloses the protein sequence of SELADIN-1 (SEQ ID NO. 1). The full length protein consists of 516 amino acid residues. The sequence is given in the one letter amino acid code.

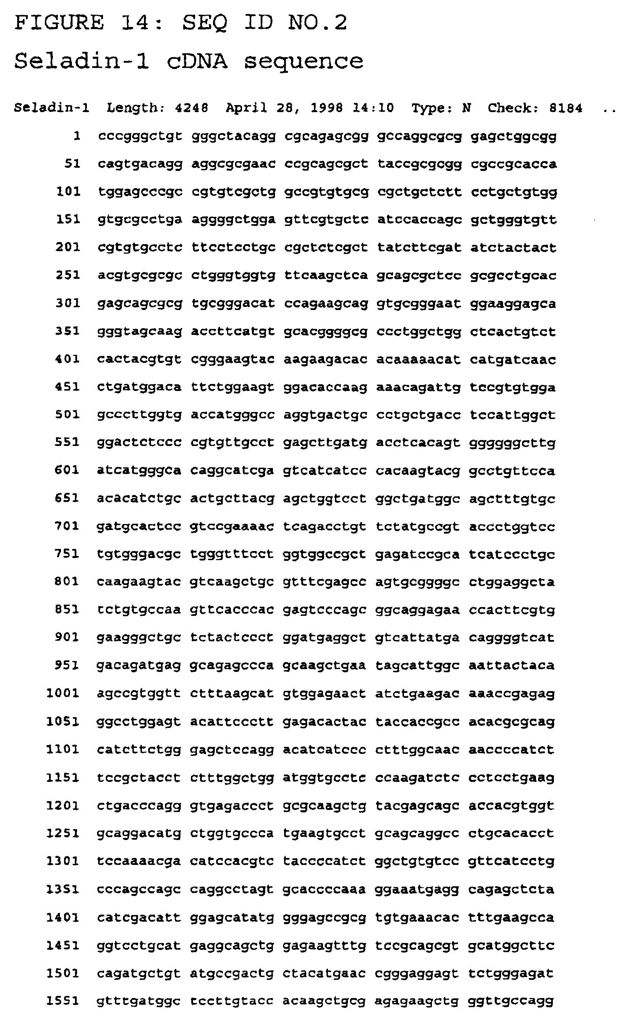

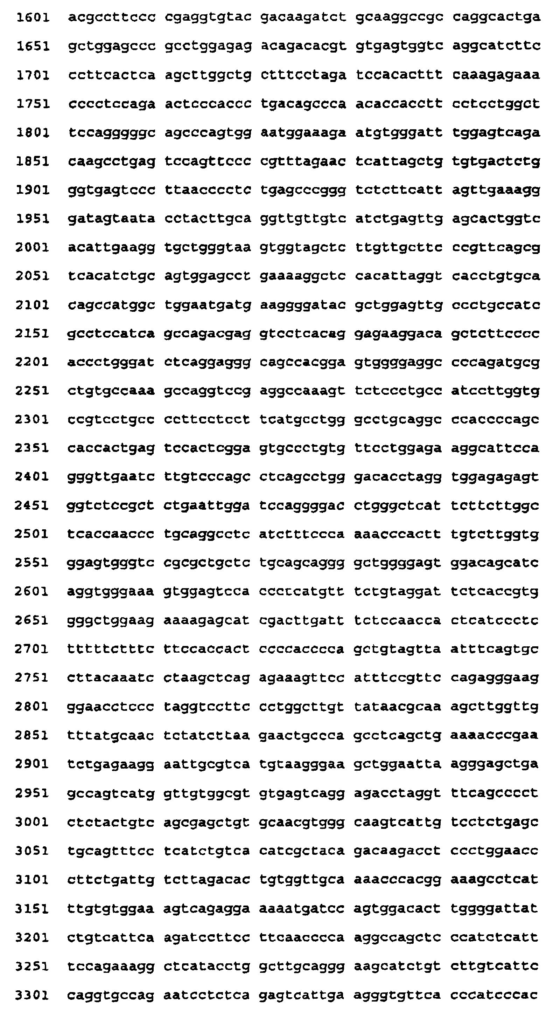

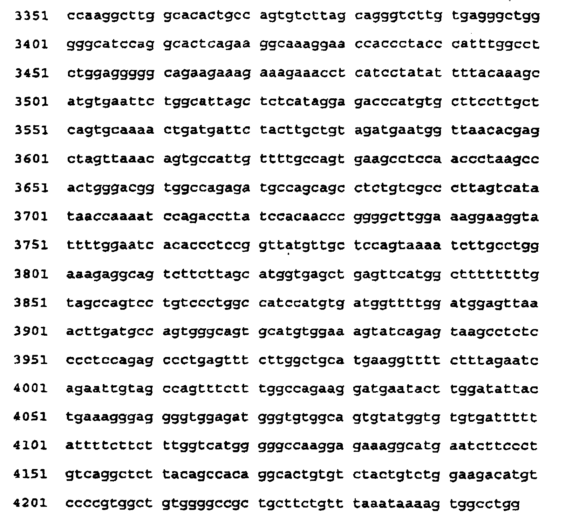

Figure 14 discloses the nucleotide sequence of the cloned *SELADIN-1* cDNA (SEQ ID NO. 2) comprising 4248 nucleotides. The coding sequence for the SELADIN-1 protein starts at nucleotide position 100 and stops at position 1648.

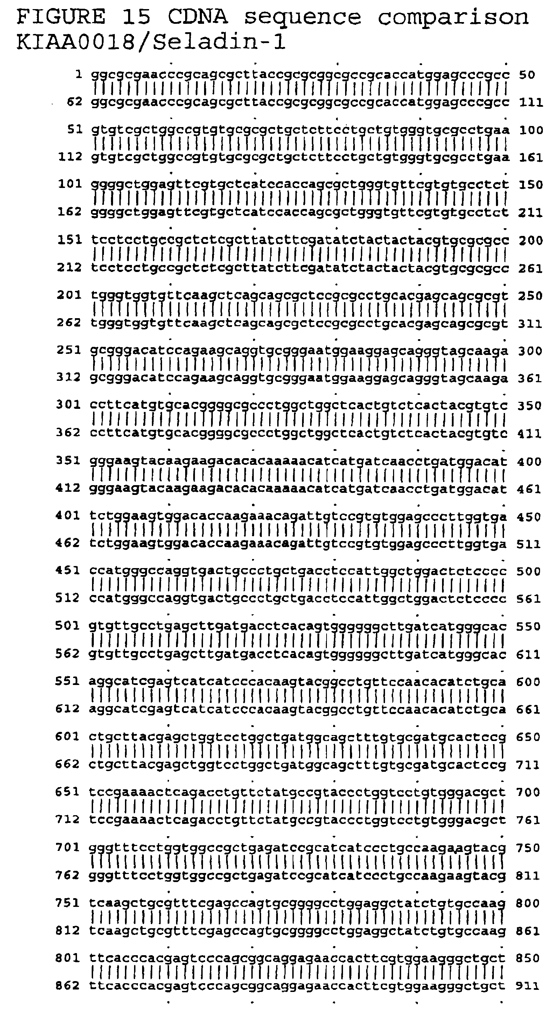

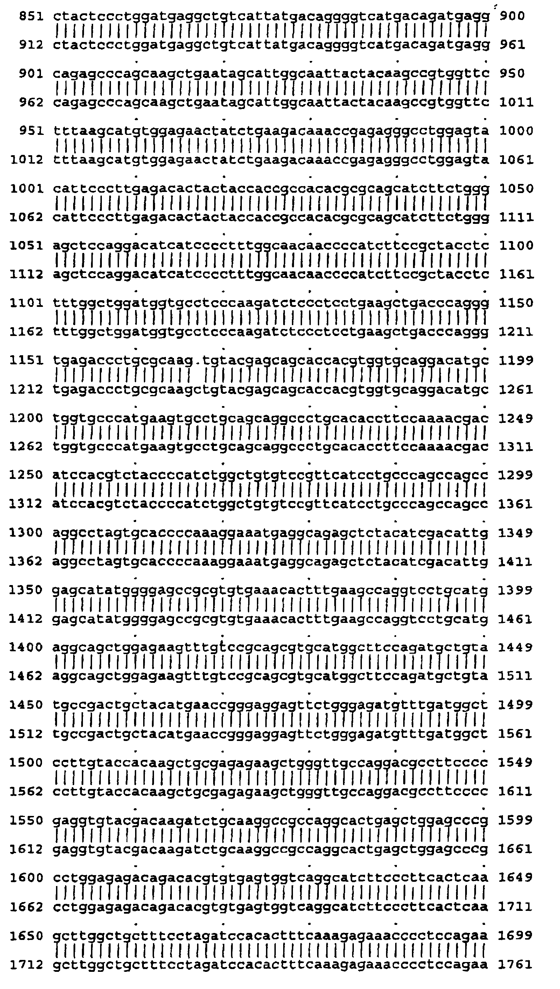

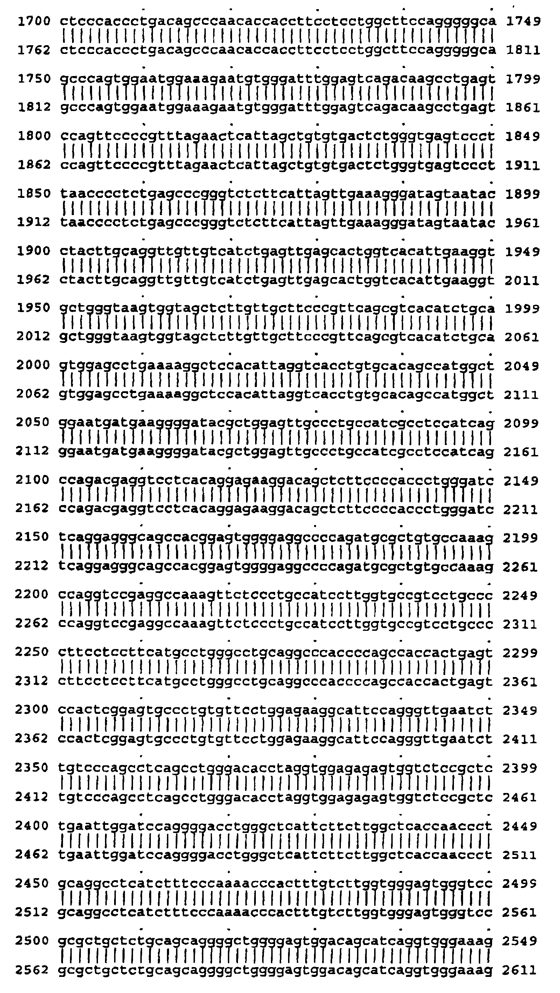

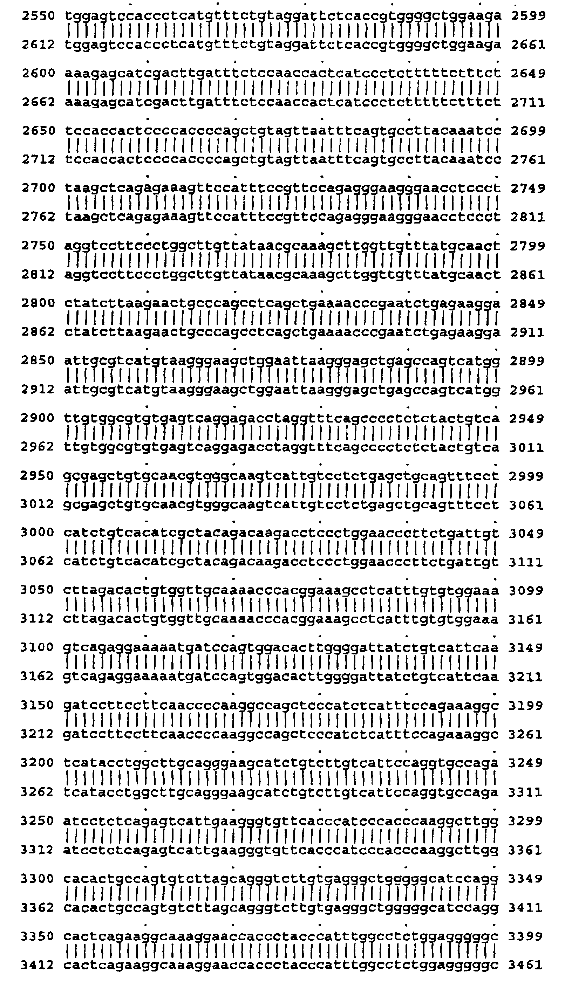

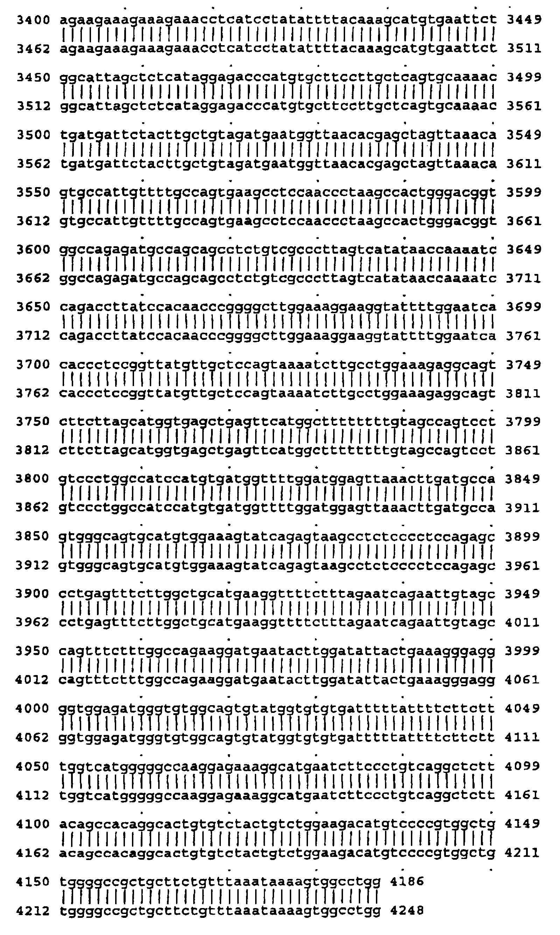

Figure 15 discloses the comparison of nucleotide sequences of the cloned *SELADIN-1* cDNA comprising 4248 nucleotides and the *KIAA0018* cDNA comprising 4186 nucleotides. A significant difference exists at position 1228 of the *SELADIN-1* sequence where a C nucleotide (C/G basepair) is missing in the *KIAA0018* sequence. This results in a frameshift in the open reading frame in the *KIAA0018* sequence relative to the *SELADIN-1* sequence. The consequence is that the translation product of the *KIAA0018* gene is 390 amino acids in length compared to 516 amino acid residues of the SELADIN-1 translation product. In addition to the difference in length, the frameshift causes a difference between the C-terminal 14 amino acids of the KIAA0018 protein and the corresponding sequence area of the SELADIN-1 polypeptide (pos. 377 - 390). The coding sequence for the SELADIN-1 protein starts at nucleotide position 100 and stops at position 1648.

### EXAMPLE I

### Post-mortem Alzheimer's disease brain tissues

Brain tissues from Alzheimer's disease patients and control subjects were removed within 6 hours of death, and immediately frozen on dry ice. Parallel sections were fixed in formaldehyde for histopathological confirmation of the diagnosis and for cell counts. Brain areas with massive neuronal cell loss as well as areas with largely preserved neurons were identified for comparisons of gene expression and stored at - 80°C until RNA extractions were performed.

### Identification of SELADIN-1 by differential display PCR

Total RNA from post-mortem brain tissues was prepared by using the RNeasy kit (Qiagen). The RNA preparations were treated with DNase I (Boehringer Mannheim) together with RNAsin (Promega) for 30 minutes, followed by phenol extraction, and ethanol precipitation. 0.2 mg of each RNA preparation were transcribed to cDNA by using Expand Reverse Transcriptase (Boehringer Mannheim) with one base ancor primers HT₁₁A, HT₁₁C and HT₁₁G. In the following PCR reaction, the cDNAs were amplified by using HT₁₁A along with the random primers HAP-5 (5'-TGCCGAAGCTTGGAGCTT-3') and HAP3-T (5'TGCCGAAGCTTTGGTCAT-3'). Taq-polymerase (AmpliTaq, Perkin Elmer Corp.), dGTP, dCTP, and dTTP (Amersham Pharmacia Biotech) and (α³⁵S)-dATP (NEN life science products) were used in a PCR protocol according to Zhao et al. The PCR products were separated on 6% polyacrylamide-urea sequencing gels that were dried subsequently on 3 mm filter paper (Whatman), and X-ray films (Dupont) were exposed for 12 hours.

### Cloning and sequencing

Differential bands were excised from the gel, boiled in water for 10 minutes, centrifuged, and cDNAs were precipitated from the supernatant fluids by using ethanol and glycogen/sodiumacetate, followed by dialysis against 10% glycerol for 1 hour through 0.025 mm filters (type VS, Millipore). The dialysates were used as templates for the reamplification reactions that were done under identical conditions as in the differential display PCR, with the exception of the initial cycle for nonspecific annealing. The resulting PCR products were separated by agarose gelelectrophoresis, purified from the gel with the QIAEXII Agarose Gel Extraction Kit (Qiagen), and cloned into the *Hind* III restriction site of pBluescript KS (Stratagene). Cloned cDNA fragments were sequenced with an ABI 377 DNA sequencer (Perkin Elmer Corp.) by using T3 and T7 primers.

### Amplification of a SELADIN-1 cDNA-fragment

A *SELADIN-1* cDNA fragment was amplified by using cDNA transcribed from human brain tissue by using RNA High Fidelity Taq-polymerase (Boehringer Mannheim) and *SELADIN-1*-specific primers for a PCR reaction with 40 cycles of annealing at 70 °C for 1 minute, and polymerization at 72 °C for 3 minutes. The PCR products were separated by agarose gel electrophoresis, purified, and cloned into the *Sma* I restriction site of pBluescript KS (Stratagene). The cloned *SELADIN-1* PCR product was sequenced, and restriction fragments were used as probes both for screening a human brain cDNA library and for probing Northern blots.

### Northern blotting

Total RNA from post-mortem human brains were prepared by using the Trizol reagent (Gibco BRL, Life Technologies), following the manufacturers instructions. 5 - 10 mg of RNA were separated in 1 % formaldehyde-containing agarose gels, and the RNA was blotted onto nylon membranes (Hybond-N⁺, Amersham). Membranes were hybridized with (α³²P)-dCTP (NEN) labeled *SELADIN-1*-specific cDNA probes that were generated by using the Megaprime DNA labelling kit (Amersham). Membranes were washed under high stringency conditions, and X-ray films were exposed for 1 to 72 hours. To control for equal loading of RNA, the identical membranes were probed with a 700 bp cDNA fragment of human glycerolaldehyd-3-phosphate dehydrogenase (*GAPDH*), or with a β-actin cDNA fragment (Clontech).

### In situ hybridization

Several *SELADIN-1*-specific cDNA probes of 650bp and of 900bp representing the initial two parts of the open reading frame were cloned in pBluescript (Stratagene) and reversely transcribed in the presence of (α³⁵S)-CTP by using the Ambion transcription kit. In situ hybridization was done with, 14mm sections of adult rat brain cut on a cryomicrotome, mounted on aminoalkylsilane-treated slides and fixed in 4 % paraformaldehyde in PBS for 5 min at room temperature. After washing for 5 min in PBS, sections were acetylated for 10 min, passed through a series of increasing ethanol grades and air dried. Prehybridizations were done in 50 % deionized formamide, 25 mM EDTA, 25 mM Pipes, pH 6.8, 0.75 M NaCl, 0.2 % SDS, 5xDenhardt's, 10 mM DTT, 250 mg/ml denatured herring sperm DNA and 250 mg/ml yeast tRNA. Hybridization of slides with RNA sense and antisense probes diluted to 2000 ― 5000 cpm/ml in the same buffer with additional 10 % dextransulphate was performed at 50°C for 12 hours. Slides were then washed four times in 4xSCC for 5 min. each, followed by an incubation for 30 min. at 37 °C with 40 mg/ml RNAseA in 0.5 M NaCl, 10 mM Tris-HCL, pH 7.5, 1mM EDTA and another 30 mm without RNAseA. Then slides were washed twice for 15 min. at 50°C in 2xSCC and dried through graded ethanols. Slides were exposed to Kodak Biomax x-ray films for 15 days and subsequently dipped in Kodak NTB-3 nuclear track emulsion and exposed for 6 weeks. After developing in Kodak D19 and fixing in Kodak Unifix, slides were counterstained with 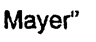 Hemalaun and coversplipped.

### Recombinant expression of SELADIN-1-EGFP fusion proteins in tissue culture

The complete coding region of *SELADIN-1* was subcloned into the N-terminus of the pEGFP-N1-expression vector (Clontech). Cos-7-cells were transfected with *EGFP* or with *SELADIN-1-EGFP* by using the SuperFect transfection reagent from Qiagen according to the manufacturers instructions. Cells were cultured in 3 cm dishes for two days. Part of the cells were stained for the Golgi-apparatus with 0.25 mM BODIPY TR ceramide (molecular probes) for one hour, the other part was treated with 250 nM of the mitochondrial stain Mito Tracker Red CM-H2Xros (Molecular probes) for 45 min. the subcellular localization of the SELADIN-1-EGFP fusion protein was analyzed by confocal laser scanning microscopy using the appropriate filter sets.

## Claims

1. An isolated nucleic acid molecule encoding a protein molecule shown in SEQ ID NO. 1.

2. A DNA molecule having a coding sequence as shown in SEQ ID NO. 2 or a coding sequence substantially the same as the coding sequence of SEQ ID NO. 2.

3. A protein molecule shown in SEQ ID NO. 1.

4. A method of diagnosing or prognosing a neurological disease in a subject comprising:
determining a level, or an activity, or both said level and said activity, of at least one substance which is selected from the group consisting of
a) a protein molecule shown in SEQ ID NO. 1,
b) a variant or fragment thereof,
c) a nucleic acid molecule encoding a protein molecule shown in SEQ ID NO. 1, a variant or fragment thereof,
d) a molecule affecting directly or indirectly a level, or an activity, or both said level and said activity, of at least one substance which is selected from the group consisting of a) to c),
e) a molecule which is affected in its level, or its activity, or both its level and activity, by at least one substance which is selected from the group consisting of a) to c)
in a sample from said subject;
and
comparing said level, or said activity, or both said level and said activity, of at least one of said substances a) to e) to a reference value representing a known disease or health status,
thereby diagnosing or prognosing said neurological disease in said subject.

5. The method according to claim 4, wherein said subjects suffers from Alzheimer's disease and related neurofibrillary disorders, or neurodegenerative states characterized by cell degeneration or cell death.

6. A method of evaluating a treatment for a neurological disease, comprising:
determining a level, or an activity, or both said level and said activity, of at least one substance which is selected from the group consisting of
a) a protein molecule shown in SEQ ID NO. 1,
b) a variant or fragment thereof,
c) a nucleic acid molecule encoding a protein molecule shown in SEQ ID NO. 1, a variant or fragment thereof,
d) a molecule affecting directly or indirectly a level, or an activity, or both said level and said activity, of at least one substance which is selected from the group consisting of a) to c),
e) a molecule which is affected in its level, or its activity, or both its level and activity, by at least one substance which is selected from the group consisting of a) to c)
in a sample from said subject;
and
comparing said level, or said activity, or both said level and said activity, of at least one of said substances a) to e) to a reference value representing a known disease or health status,
thereby evaluating said treatment for said neurological disease.

7. An agent which directly or indirectly affects an activity, or level, or both said activity or level, of at least one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and activity, of at least one substance which is selected from the group consisting of a) to c),
e) a molecule which is affected in its level, or its activity, or both its level and activity, by at least one substance which is selected from the group consisting of a) to c).

8. A medicament comprising an agent according to claim 7.

9. An agent for treating or preventing a neurological disease, which agent directly or indirectly affects an activity, or level, or both said activity or level, of at least one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and activity of at least one substance which is selected from the group consisting of a) to c),
e) a molecule which is affected in its level, or its activity, or both its level and activity, by at least one substance which is selected from the group consisting of a) to c).

10. A medicament for treating or preventing a neurological disease comprising an agent according to claim 9.

11. Use of an agent for treating or preventing a neurological disease, which agent directly or indirectly affects an activity, or level, or both said activity or level, of at least one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and activity of at least one substance which is selected from the group consisting of a) to c),
e) a molecule which is affected in its level, or its activity, or both its level and activity, by at least one substance which is selected from the group consisting of a) to c).

12. A method for identifying an agent that directly or or indirectly affects an activity, or level, or both said activity or level, of at least one substance which is selected from the group consisting of
a) a protein molecule shown in SEQ ID NO.1,
b) a variant or fragment thereof,
c) a nucleic acid molecule encoding a protein molecule shown in SEQ ID NO.1, a variant or fragment thereof,
d) a molecule affecting directly or indirectly a level, or an activity, or both said level and activity of at least one substance which is selected from the group consisting of a) to c),
e) a molecule which is affected in its level, or its activity, or both its level and activity, by at least one substance which is selected from the group consisting of a) to c) comprising the steps of:
(i) providing a sample containing at least one substance which is selected from the group consisting of a) to e);
(ii) contacting said sample with at least one agent;
(iii) comparing an activity, or level, or both said activity and level, of at least one of said substances before and after said contacting.
